# EUROPEAN PATENT APPLICATION

(11) **EP 2 184 064 A2**
(43) Date of publication of application: **12.05.2010**
(21) Application number: 07861047.4
(22) Date of filing: 15.11.2007
(51) Int. Cl.: A61K 31/404, A61K 31/4045, A61K 31/407, A61K 31/4178, A61K 31/4184, A61K 31/4196, A61K 31/433, A61K 31/437, A61K 31/4439, A61K 31/454, A61K 31/4748, A61K 31/497, A61K 31/4985, A61K 31/52, A61K 31/522, A61K 31/529, A61K 31/5513, A61K 31/55, C07D 209/14, C07D 209/18, C07C 209/22

(54) **LIGANDS OF 5-HT6 RECEPTORS, A PHARMACEUTICAL COMPOSITION, METHOD FOR THE PRODUCTION AND USE THEREOF**

(30) Priority: 16.11.2006 RU 2006140353
(71) Applicant: Alla Chem, LLC., Carson City, NV 89701 (US); IVASHCHENKO, Andrey Alexandrovich, Moscow 127576 (RU)
(72) Inventor: IVASHCHENKO, Andrey Alexandrovich, Moscow 127576 (RU); IVASHCHENKO, Alexandr Vasilievich, Encinitas, CA 92024 (US); TKACHENKO, Sergey Yevgenievich, Moskovskaya obl., 142432 (RU); OKUN, Ilya Matusovich, San-Diego, CA 92121 (US); SAVCHUK, Nikolay Filippovich, Moscow, 117420 (RU)
(74) Representative: Jeck, Anton
(86) International application number: PCT/RU2007/000624
(87) International publication number: WO 2008/060190

(57) **Abstract**

The invention relates to novel ligands of 5-HT₆ receptor, to a pharmaceutical composition containing said novel ligands of 5-HT₆ receptor as active component and to novel medicaments used for humans and warm-blooded animals for treating diseases and conditions of central nervous system, in pathogenesis of which neuromediator systems induced by 5-HT₆ receptors are playing an essential role.

Azaheterocyclic compounds of the general formula 1 or racemates, or optical or geometrical isomers, or pharmaceutically acceptable salts and/or hydrates thereof are used as 5-HT₆ ligands. 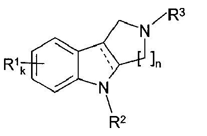 Wherein R2 and R3 independently of each other represent an amino group substituent selected from hydrogen; substituted carbonyl; substituted aminocarbonyl; substituted aminothiocarbonyl; substituted sulphonyl; C₁-C₅-alkyl optionally substituted by: C₆-C₁₀-arylaminocarbonyl, heterocyclyl, C₆-C₁₀-arylaminocarbonyl, C₆-C₁₀-arylaminothiocarbonyl, C₅-C₁₀-azaheteroaryl, optionally substituted carboxyl, nitryl group, optionally substituted aryl; R¹ₖ represents from 1 to 3 substituents of cyclic system, independent of each other and selected from hydrogen, optionally substituted C₁-C₅-alkyl, C₁-C₅-alkoxy, C₁-C₅-alkenyl, C₁-C₅-alkynyl, halogen, trifluoromethyl, CN-group, carboxyl, optionally substituted aryl, optionally substituted heterocyclyl, substituted sulfonyl, optionally substituted carboxyl; the solid line accompanied by the dotted line (---) represents a single or a double bond; n=1,2 or 3.

## Description

### Field of the invention

The invention relates to the novel ligands of serotonin 5-HT₆ receptors, to pharmaceutical composition, comprising novel ligands of serotonin 5-HT₆ receptors as an active ingredient, to novel medicaments useful for treatment of diseases and conditions of the central nervous system (CNS) in pathogenesis of which neuromediator systems induced by 5-HT₆ receptors are playing an essential role.

### Background of the invention

The development of effective remedies for treatment and prophylaxis of Alzheimer's disease (AD) and other neurodegenerative diseases has being conducted for many years, however, up to now the main tendency of the searching is an attempt to correct pharmacologically the cholinergic deficit [Mayeux R., Sano M. Treatment of Alzhaimer's disease. N. Engl. J. Med. 1999; 341:1670-1679], that led to the development of the whole number of medicaments based upon acetylcholinesterase inhibitors (Tacrine, Amiridinum, Aricept and so on), exhibiting , however, a rather limited clinical applicability. In this context the searching of highly effective remedies for treatment and early detection of AD with an essentially new mechanism of action becomes more and more actual problem [Jellinger K.A. Alzheimer 100 - highlights in the history of Alzheimer research. J Neural Transm. 2006 Oct 13; [Epub ahead of print].

In this respect one of the most perspective approaches is the utilization of effective and selective antagonists of serotonin 5-HT₆ receptors for treatment of diseases associated with CNS, in particular, AD and others neurodegenerative diseases [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. At mammals these receptors are localised exclusively in the central nerves system (CNS), and mainly in brain regions responsible for training and memory [Ge'rard C., Martres M.-P., Lefe`vre K., Miquel M.-C., Verge' D., Lanfumey L., Doucet E., Hamon M., El Mestikawy S. Immuno-localisation of serotonin 5-HT6 receptor-like material in the rat central nervous system. Brain Research. 1997; 746:207-219]. Besides, it has been shown [Dawson L.A., Nguyen H.Q., Li P. The 5-HT(6) receptor antagonist SB-271046 selectively enhances excitatory neurotransmission in the rat frontal cortex and hippocampus. Neuropsychopharmacology. 2001; 25:662-668], that 5-HT₆ receptors are the modulators of a number of neuromediator systems including cholinergic, noradrenergic, glutamatergic and dopaminergic. Taking into account the fundamental role of these systems in normal cognitive processes and also their disfunction at neurodegeneration, an exclusive role of 5-HT₆ receptors in forming normal or "pathological" memory becomes obvious. In a great number of current publications it has been shown that blocking of 5-HT₆ receptors leads to a considerable enhancement of memory consolidation in various animal models of training-memorising-reproduction [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100. Riemer C., Borroni E., Levet-Trafit B., Martin J.R., Poli S., Porter R.H., Bos M. Influence of the 5-HT6 receptor on acetylcholine release in the cortex: pharmacological characterization of 4-(2-bromo-6-pyrrolidin-1-ylpyridine-4-sulfonyl)phenylamine, a potent and selective 5-HT(6) receptor antagonist. J. Med. Chem. 2003; 46:1273-1276. King M.V., Woolley M.L., Topham I.A., Sleight A.J., Marsden C.A., Fone K.C. 5-HT(6) receptor antagonists reverse delay-dependent deficits in novel object discrimination by enhancing consolidation e an effect sensitive to NMDA receptor antagonism. Neuropharmacology 2004; 47:195-204]. It has also been shown a considerable enhancement of cognitive functions in aged rats under the action of 5-HT₆ receptor antagonists in the model of Morrison's water maze. [Foley A.G., Murphy K.J., Hirst W.D., Gallagher H.C., Hagan J.J., Upton N., Walsh F.S., Regan C.M. The 5-HT(6) receptor antagonist SB-271046 reverses scopolamine-disrupted consolidation of a passive avoidance task and ameliorates spatial task deficits in aged rats. Neuropsychopharmacology. 2004; 29:93-100]. Recently not only the more thorough understanding of 5-HT₆ receptor's role in cognitive processes, but more accurate conceptions concerning possible pharmacophoric properties of their antagonists were achieved [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299]. This resulted in preparation of highly affine selective ligands ("molecular tools"), and then clinical candidates. Now four antagonists of 5-NT₆ receptors as potential ingredients for treatment of AD and other cognitive diseases are at various phases of clinical investigation [http://integrity.prous.com].

Another attractive property of 5-HT₆ receptor antagonists is their ability to suppress appetite that can lead to development on their basis of essentially novel remidies for lowering of overweight and obesity [Vicker S.P., Dourish C.T. Serotonin receptor ligands and the treatment of obesity. Curr. Opin. Investig. Drugs. 2004; 5:377-388]. This effect has been confirmed in many investigations [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299. Davies S.L. Drug discovery targets: 5-HT6 receptor. Drug Future. 2005; 30:479-495], the mechanism of their action is based upon suppression of γ-aminobutyric acid signaling by 5-HT₆ receptor antagonists and increasing of α-melanocyte-stimulating hormone emission, that, finally, results in lowering of food consumption [Woolley M.L. 5-ht6 receptors. Curr. Drug Targets CNS Neurol. Disord. 2004; 3:59-79].

5-HT₆ Receptor antagonists are particularly perspective as potential remedies for AD treatment, other CNS diseases and obesity because of their exceptional distribution within some regions of brain that eliminates the possibility of manifestation of practically any peripheral side effects.

It should be noted that in scientific and patent literature there are examples of 5-HT₆ receptor ligands, belonging to various class of organic compounds [Holenz J., Pauwels P.J., Diaz J.L., Merce R., Codony X., Buschmann H. Medicinal chemistry strategies to 5-HT6 receptor ligands as potential cognitive enhancers and antiobesity agents. Drug Disc. Today. 2006; 11:283-299], among them effective antagonists of 5-HT₆ receptor were discovered, such as (**a**)-1-benzenesulfonyl- and (**b**)- 1-(4-aminobenzenesulfonyl)-3-(dimethylaminomethyl)indole [M.R. Pullagurla, M. Dukat, V. Setola, B. Roth and R.A. Glennon. N1-Benzenesulfonylgramine and N1-Benzenesulfonylskatole: Novel 5-HT6 Receptor Ligand Templates. Bioorganic & Medicinal Chemistry Letters, 2003, 13, 3355-3359].

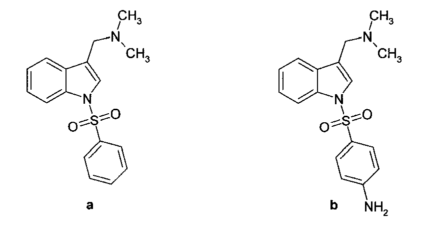

As a result of the conducted investigation directed to the development of novel biologically active compounds the inventors discovered that annelated azaheterocyclic compounds comprising 3-(aminomethyl)indole fragment exhibit the ability to block 5-HT₆ receptors effectively.

More specifically, the present invention refers to the novel ligands of 5-hydroxytriptamine (5-HT₆) receptor, which are annelated azaheterocyclic compounds comprising 3-(aminomethyl)indole fragment; to pharmaceutical composition comprising the novel antagonists of 5-HT₆ receptor as active ingredients; and to the method for preparation of this composition; to the medicine for treatment and prophylaxis of various diseases of CNS, such as AD, Parkinson's disease, Huntington's chorea, lathyrism, amyotrophic lateral sclerosis, schizophrenia and other neurologic and neurodegenerative diseases and various mental disorders; and for overweight lowering and treatment of obesity.

### Disclosure of the invention

In the context of the present invention, the terms are generally defined as follows:
**"Azaheterocycle"** means an aromatic or nonaromatic mono- or polycyclic system with at least one nitrogen atom the meanings of which are defined in this section. Azaheterocycle may have one or more "cyclic system substituents".
**"Aliphatic"** radical means the radical derived at removal of hydrogen atom from nonaromatic C-H bond. Aliphatic radical may additionally contain any substituens - aliphatic or aromatic radicals, the meanings of which are defined in this section. The representatives of aliphatic radicals include: alkyl, alkenyl, alkynyl, cycloalkyl, cycloalkenyl, heterocyclyl, heterocyclenyl, aralkenyl, aralkyloxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, annelated arylcycloalkyl, annelated heteroarylcycloalkyl, annelated arylcycloalkenyl, annelated heteroarylcycloalkenyl, annelated arylheterocyclyl, annelated heteroarylheterocyclyl, annelated arylheterocyclenyl, annelated heteroarylheterocyclenyl.
"Alkenyl" means an aliphatic straight- or branched- hydrocarbon group with 2 - 7 carbon atoms including C=C double bond. "Branched" means that one or several lower alkyl substituents, such as methyl, ethyl or propyl are attached to the straight alkenyl chain. Alkyl substituent may have one or more substituents such as: halogen, alkenyloxy, cycloalkyl, cyano; hydroxy, alkoxy, carboxy, alkynyloxy, aralkoxy, aryloxy, aryloxycarbonyl, alkylthio, heteroaralkyloxy, heterocyclyl, heterocyclylalkyloxy, alkoxycarbonyl, aralkoxycarbonyl, heteroaralkoxycarbonyl or G¹G²N-, G¹G²NC(=O)-, G¹G²NSO₂-, where G¹ and G² independently of each other represent hydrogen atom, alkyl, aryl, aralkyl, heteroaralkyl, heterocyclyl or heteroaryl, or G¹ and G² together with the N-atom they are attached to, form through G¹ and G² 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, benzyloxycarbonylmethyl, and pyridylmethyloxycarbonylmethyl. The preferred alkenyl groups are ethenyl, propenyl, n-butenyl, iso-butenyl, 3-methylbuten-2-yl, n-pentenyl and cyclohexylbutenyl.
**"Alkenyloxy"** means alkenyl-O-group wherein alkenyl is defined in this section. The preferred alkenyloxy groups are allyloxy and 3-butenyloxy.
**"Alkenyloxyalkyl"** means alkenyl-O-alkyl group wherein alkyl and alkenyl are defined in this section.
**"Alkyl"** means an aliphatic hydrocarbon straight or branched group with 1-12 carbon atoms. Branched means that the alkyl chain has one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NC(=S)-, G¹G²NSO₂-, where G¹ and G² independently of each other represent hydrogen atom, alkyl, aryl, aralkyl, heteroaralkyl, heterocyclyl or heteroaryl, or G¹ and G² together with the N-atom, they are attached to, form through G¹ and G² 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl and pyridilmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G²NC(=O)-, annelated arylheterocyclenyl, annelated arylheterocyclyl.
**"Alkyloxyalkyl"** means alkyl-O-alkyl group, wherein alkyl groups are independent of each other and defined in this section. The preferred alkyloxyalkyl groups are methoxyethyl, ethoxymethyl, n-buthoxymethyl, methoxypropyl and iso-propyloxyethyl.
**"Alkylthio"** means alkyl-S group wherein alkyl group is defined in this section.
**"Alkoxy"** means alkyl-O-group, wherein alkyl is defined in this section. The preferred alkoxy groups are methoxy, ethoxy, n-propoxy, iso-propoxy and n-butoxy.
**"Alkoxycarbonyl"** means alkyl-O-C(=O)-group, wherein alkyl is defined in this section. The preferred alkoxycarbonyl groups are methoxycarbonyl, ethoxycarbonyl, and tert-butoxycarbonyl.
**"Alkoxycarbonylalkyl"** means alkyl-O-C(=O)-alkyl-group, wherein alkyl is defined in this section. The preferred alkoxycarbonylalkyl groups are methoxycarbonylmethyl and ethoxycarbonylmethyl; and methoxycarbonylethyl and ethoxycarbonylethyl.
**"Amino group"**means G¹G²N-group substituted or not by "amino group substituent" G¹ and G², the meanings of which are defined in this section, for example, amino (NH₂), methylamino, diethylamino, pyrrolidino, morpholino, benzylamino or phenethylamino.
**"Annelated cycle" (condensed cycle)** means bi- or poly-cyclic system wherein annelated cycle and a cycle or polycycle with which it is annelated have at least two common atoms.
**"Annelated arylheterocycloalkenyl"** means annelated aryl and heterocycloalkenyl, the meanings of which are defined in this section. Annelated arylheterocycloalkenyl could be attached to any other fragment via any atom of its own system. Prefix "aza", "oxa" or "thia" before "heterocycloalkenyl" means that N, O or S atoms are introduced in the appropriate cyclic fragment. Annelated arylheterocycloalkenyl may have one or more "cyclic system substituens" of the same or different structure. N- and S-atoms of the heterocycloalkenyl fragment could be oxidized to N-oxide, S-oxide or S-dioxide. Indolinyl, 1H-2-oxoquinolinyl, 2H-1-oxoisoquinolinyl, 1,2-dihydroquinolinyl and others are the representatives of annelated arylheterocycloalkenyl.
**"Annelated arylheterocycloalkyl"** means annelated aryl and heterocycloalkyl, the meanings of which are defined in this section. Annelated arylheterocycloalkyl could be attached to any other fragment via any atom of its own system. Prefix "aza", "oxa" or "thia" before "heterocycloalkyl" means that N, O or S atoms are introduced in the appropriate cyclic fragment. Annelated arylheterocycloalkyl may have one or more "cyclic system substituens" of the same or different structure. N- and S-atoms of the heterocycloalkyl fragment could be oxidized to N-oxide, S-oxide and S-dioxide. Indolyl, 1,2,3,4-tetrahydroisoquinolinyl, 1,3-benzodioxolyl and others are the representatives of annelated arylheterocycloalkyl.
**"Annelated arylcycloalkenyl"**means annelated aryl and cycloalkenyl, the meanings of which are defined in this section. Annelated arylcycloalkenyl could be attached to any other fragment via any atom of its own system. Annelated arylcycloalkenyl may have one or more "cyclic system substituents" of the same or different structure. 1,2-Dihydronaphthalenyl, indenyl and others are the representatives of annelated arylcycloalkenyl.
**"Annelated arylcycloalkyl"** means annelated aryl and cycloalkyl, the meanings of which are defined in this section. Annelated arylcycloalkyl could be attached to any other fragment via any atom of its own system. Annelated arylcycloalkyl may have one or more "cyclic system substituens" of the same or different structure. Indaninyl, 1,2,3,4-tetrahydronaphthyl, 5,6,7,8-tetrahydronaphth-1-yl and others are the representatives of annelated arylcycloalkyl.
**"Annelated heteroarylcycloalkenyl"** means annelated heteroaryl and cycloalkenyl, the meanings of which are defined in this section. Annelated heteroarylcycloalkenyl could be attached to any other fragment via any atom of its own system. Prefix "aza", "oxa" or "thia" before "heteroaryl" means that N, O or S atoms are introduced in the cyclic system. Annelated heteroarylcycloalkenyl may have one or more "cyclic system substituents" of the same or different structure. N-Atom of the heteroaryl ring could be oxidized to N-oxide. 5,6-Dihydroquinolinyl, 5,6-dihydroisoquinolinyl, 4,5-dihydro-1H-benzimidazolyl and others are the representatives of annelated heteroarylcycloalkenyl.
**"Annelated heteroarylcycloalkyl"** means annelated heteroaryl and cycloalkyl, the meanings of which are defined in this section. Annelated heteroarylcycloalkyl could be attached to any other fragment via any atom of its own system. Prefix "aza", "oxa" or "thia" before "heteroaryl" means that N, O or S atoms are introduced in the appropriate cyclic fragment. Annelated heteroarylcycloalkyl may have one or more "cyclic system substituents" of the same or different structure. N-Atom of the heteroaryl part of the molecule could be oxidized to N-oxide. 5,6,7,8-Tetrahydroquinolinyl, 5,6,7,8-tetrahydroisoquinolinyl, 4,5,6,7-tetrahydro-1H-benzimidazolyl and others are the representatives of annelated heteroarylcycloalkyles.
**"Annelated heteroarylheterocyclenyl"** means annelated heteroaryl and heterocyclenyl, the meanings of which are defined in this section. Annelated heteroarylheterocyclenyl could be attached to any other fragment via any atom of its own system. Prefix "aza", "oxa" or "thia" before "heteroaryl" means that N, O or S atoms are introduced in the appropriate cyclic fragment. Annelated heteroarylheterocyclenyl may have one or more "cyclic system substituents" of the same or different structure. N-Atom of heteroaryl fragment could be oxidized to N-oxide. N- And S-atoms of the heterocyclenyl fragment could be oxidized to N-oxide, S-oxide and S-dioxide. 1,2-Dihydro[2,7]naphthiridinyl, 7,8-dihydro[1,7]naphthiridinyl, 6,7-dihydro-3H-imidazo[4,5-c]pyridyl and others are the representatives of annelated heteroarylheterocyclenyl
**"Annelated heteroarylheterocyclyl"** means annelated heteroaryl and heterocyclyl, the meanings of which are defined in this section. Annelated heteroarylheterocyclyl could be attached to any other fragment via any atom of its own system. Prefix "aza", "oxa" or "thia" before "heteroaryl" means that N, O or S atoms are introduced in the cyclic system. Annelated heteroarylheterocyclyl may have one or more "cyclic system substituents" of the same or different structure. N-Atom of the heteroaryl fragment could be oxidized to N-oxide. N- and S-atoms of heterocyclyl fragment could be oxidized to N-oxide, S-oxide and S-dioxide. 2,3-Dihydro-1H-pyrrolo[3,4-b]quinolin-2-yl, 2,3-dihydro-1H-pyrrolo[3,4-b]indol-2-yl, 1,2,3,4-tetrahydro[1,5]naphthiridinyl and others are the representatives of annelated heteroarylheterocyclyl.
**"Aralkenyl"** means aryl-alkenyl group, wherein the meanings of aryl and alkenyl are defined in this section. For example, 2-phenethenyl is aralkenyl group.
**"Aralkyl"** means alkyl group substituted by one or more aryl groups, wherein the meanings of aryl and alkyl are defined in this section. For example, benzyl, 2,2-diphenylethyl or phenethyl are aralkyl groups.
**"Aralkylamino"** means aryl-alkyl-NH-group, wherein the meanings of aryl and alkyl are defined in this section.
**"Aralkylsulfinyl"** means aralkyl-SO-group, wherein the meaning of aralkyl is defined in this section.
**"Aralkylsulfonyl"** means aralkyl-SO₂-group, wherein the meaning of aralkyl is defined in this section.
**"Aralkylthio"** means aralkyl-S-group, wherein the meaning of aralkyl is defined in this section.
**"Aralkoxy"** means aralkyl-O-group, wherein the meaning of aralkyl is defined in this section. For example, benzyloxy or 1- or 2-naphthylenmethoxy are aralkoxy groups.
**"Aralkoxyalkyl"** means aralkyl-O-alkyl-group, wherein the meanings of aralkyl and alkyl are defined in this section. For example, benzyloxyethyl is aralkyl-O-alkyl group.
**"Aralkoxycarbonyl"** means aralkyl-O-C(=O)-group, wherein the meaning of aralkyl is defined in this section. Benzyloxycarbonyl is an example of aralkoxycarbonyl group.
**"Aralkoxycarbonylalkyl"** means aralkyl-O-C(=O)-alkyl-group, wherein the meanings of aralkyl and alkyl are defined in this section. Benzyloxycarbonylmethyl or benzyloxycarbonylethyl are examples of aralkoxycarbonylalkyl groups.
**"Aryl"** means aromatic mono- or polycyclic system with 6 - 14 carbon atoms, preferably from 6 to 10 C-atoms. Aryl may have one or more "cyclic system substituents" of the same or different structure. Phenyl or naphthyl, substituted phenyl or substituted naphthyl are the representatives of aryl groups. Aryl could be annelated with nonaromatic cyclic system or heterocycle.
**"Arylcarbamoyl"** means aryl-NHC(=O)-group, wherein the meaning of aryl is defined in this section.
**"Aryloxy"** means aryl-O-group, wherein the meaning of aryl is defined in this section. Phenoxy and 2-naphthyloxy are the representatives of aryloxy group.
**"Aryloxycarbonyl"** means aryl-O-C(=O)-group, wherein the meaning of aryl is defined in this section. Phenoxycarbonyl and 2-naphthoxycarbonyl are the representatives of aryloxycarbonyl groups.
**"Arylsulfinyl"** means aryl-SO-group, wherein the meaning of aryl is defined in this section.
**"Arylsulfonyl"** means aryl-SO₂-group, wherein the meaning of aryl is defined in this section.
**"Arylthio"** means aryl-S-group, wherein the meaning of aryl is defined in this section. Phenylthio and 2-naphthylthio are the representatives of arylthio groups.
**"Aroylamino"** means aroyl-NH-group, wherein the meaning of aroyl is defined in this section.
"Aroyl" means aryl-C(=O)-group, in which the meaning of aryl is defined in this section. Benzoyl, 1- and 2-naphthoyl are the representatives of aroyl groups.
**"Aromatic** radical" means a radical derived at removal of hydrogen atom from aromatic C-H bond. "Aromatic" radical implies aryl and heteroaryl cycles, the meaning of which are defined in this section. Aryl and heteroaryl cycles may additionally contain substituents, such as aliphatic and aromatic radicals, the meaning of which are defined in this section. Aryl, annelated cycloalkenylaryl, annelated cycloalkylaryl, annelated heterocyclylaryl, annelated heterocyclenylaryl, heteroaryl, annelated cycloalkylheteroaryl, annelated cycloalkenylheteroaryl, annelated heterocyclenylheteroaryl and annelated heterocyclylheteroaryl are the representatives of aromatic radicals.
**"Aromatic cycle"** means a plane cyclic system, in which all atoms take part in the formation of a common conjugation system comprising, according to Hückel rule, (4n + 2) π-electrons (n is a whole nonnegative number). Benzene, naphthalene, anthracene and others are the representatives of aromatic cycles. In the case of "heteroaromatic cycles" π-electrons and p-electrons of heteroatoms participate in the conjugation, so that their total number is equal to (4n + 2) as well. Pyridine, thiophene, pyrrole, furan, thiazole and others are the representatives of such cycles. Aromatic cycle may have one or more "cyclic system substituents" and could be annelated to nonaromatic cycle, heteroaromatic or heterocyclic system.
**"Acyl"** means H-C(=O)-, alkyl-C(=O)-, cycloalkyl-C(=O)-, heterocyclyl-C(=O)-, heterocyclylalkyl-C(=O)-, aryl-C(=O)-, arylalkyl-C(=O)-, heteroaryl-C(=O)-, heteroarylalkyl-C(=O)-groups, in which alkyl-, cycloalkyl-, heterocyclyl-, heterocyclylalkyl-, aryl-, arylallcyl-, heteroaryl-, heteroarylalkyl are defined in this section.
**"Acylamino"**means acyl-NH-group, wherein the meaning of acyl is defined in this section.
**"1,2-Vinyl radical"** means -CH=CH-group with one or more "alkyl substituents" of the same or different structure, the meanings of which are defined in this section.
**"Halogen"** means fluorine, chlorine, bromine and iodine. Preference is given to fluorine, chlorine and bromine.
**"Heteroannelated cycle"** means that the cycle attached (annelated or condenced) to another cycle or polycycle contains at least one heteroatom.
**"Heteroaralkenyl"** means heteroarylalkenyl-group, wherein the meanings of heteroaryl and alkenyl are defined in this section. Preferably, heteroarylalkenyl comprises the lower alkenyl group. 4-Pyridylvinyl, thienylethenyl, imidazolylethenyl, pyrazinylethenyl and others are the representatives of heteroarylalkenyl radical.
**"Heteroaralkyl"** means heteroarylalkyl-group, wherein heteroaryl and alkyl are defined in this section. Pyridylmethyl, thienylmethyl, furylmethyl, imidazolylmethyl, pyrazinylmethyl and others are the representatives of heteroaralkyl radicals.
**"Heteroaralkyloxy"** means heteroarylalkyl-O-group, wherein the meaning of heteroarylalkyl is defined in this section. 4-Pyridylmethyloxy, 2-thienylmethyloxy and others are the representatives of heteroaralkyloxy groups.
**"Heteroaryl"** means aromatic mono- or polycyclic system with 5 - 14 carbon atoms, preferably from 5 to 10, in which one or more carbon atoms are substituted by one or more heteroatoms, such as N, S or O. Prefix "aza", "oxa" or"thia" before "heteroaryl" means that atoms N, O or S are introduced in the appropriate cyclic fragment. N-Atom of heteroaryl cycle could be oxidized to N-oxide. Heteroaryl may have one or more "cyclic system sustituents" of the same or different structure. Pyrrolyl, furanyl, thienyl, pyridyl, pyrazinyl, pyrimidinyl, isoxazolyl, isothiazolyl, tetrazolyl, oxazolyl, thiazolyl, pyrazolyl, furazanyl, triazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzoimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl and others are the representatives of heteroaryl radicals.
**"Heteroarylsulfonylcarbamoyl"** means heteroaryl-SO₂-NH-C(=O)-group, wherein heteroaryl is defined in this section.
**"Heteroaroyl"-** means heteroaryl-C(=O)-group, wherein the meaning of heteroaryl is defined in this section. Nicotinoyl, thienoyl, pyrazoloyl and others are the representatives of heteroaroyl groups.
**"Heterocycle"** means aromatic or nonaromatic, monocyclic or polycyclic system comprising in the cycle, at least, one heteroatom, the meanings of which are defined in this section. The preferred heteroatoms are N, O, and S. A heterocycle may have one or more "cyclic system substituents".
**"Heterocyclenyl"** means nonaromatic mono- or polycyclic system including from 3 to 13 carbon atoms, preferably from 5 to 13 carbon atoms in which one or more carbon atoms are replaced by heteroatoms such as N, O or S and which comprises, at least, one -C=C- or -C=N-double bond. Prefix "aza", "oxa" or "thia" before "heterocyclenyl" means that atoms N, O or S are introduced in the appropriate cyclic fragment. Heterocyclenyl may have one or more "cyclic system substituens" of the same or different structure. N- and S-atoms of the heterocyclenyl fragment could be oxidized to N-oxide, S-oxide and S-dioxide. 1,2,3,4-Tetrahydropyridinyl, 1,2-dihydropyridinyl, 1,4-dihydropyridinyl, 2-pyrrolinyl, 3-pyrrolinyl, 2-imidazolyl, 2-pyrazolinyl, dihydrofuranyl, dihydrothiophenyl and others are examples of heterocyclenyl.
**"Heterocyclyl"** means a radical derived from heterocycle.
**"Heterocyclyloxy"** means heterocyclyl-O- group wherein the meaning heterocyclyl is defined in this section.
**"Hydrate"** means stoichiometric or nonstoichiometric compositions of the compounds or their salts with water.
**"Hydroxyalkyl"** means an HO-alkyl-group wherein the meaning alkyl is defined in this section.
**"Substituent"** means a chemical radical attached to a scaffold (fragment), for example, "alkyl substituent", "amino group substituent", "carbamoyl substituent" and "cyclic system substituent", the meanings of which are defined in this section..
**"Alkyl group substituent"** means a substituent attached to alkyl or alkenyl group, the meanings of which are defined in this section. It is selected from hydrogen, alkyl, halogen, alkenyloxy, cycloalkyl, aryl, heteroaryl, hetrocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonyl, heteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or G¹G²N-, G¹G²NC(=O)-, G¹G²NSO₂-, where G¹ and G² independently of each other represent hydrogen atom, alkyl, aryl, aralkyl, heteroaralkyl, heterocyclyl or heteroaryl, or G¹ and G² together with the N-atom they are attached to via G¹ and G² form 4-7-membered heterocyclyl or heterocyclenyl. The preferred alkyl groups are methyl, trifluoromethyl, cyclopropylmethyl, cyclopentylmethyl, ethyl, n-propyl, iso-propyl, n-butyl, tert-butyl, n-pentyl, 3-pentyl, methoxyethyl, carboxymethyl, methoxycarbonylmethyl, ethoxycarbonylmethyl, benzyloxycarbonylmethyl, methoxycarbonylmethyl, and pyridylmethyloxycarbonylmethyl. The preferred "alkyl group substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or G¹G²N-, G¹G²NC(=O)-, annelated arylheterocyclenyl, annelated arylheterocyclyl. The meanings of "alkyl group substituents" are defined in this section.
**"Amino group substituent"** means a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl.
**"Carbamoyl substituent"** means a substituent attached to carbamoyl group, the meaning of which is defined in this section. Carbamoyl substituent could be selected from hydrogen, alkyl, cycloalkyl, aryl, heteroaryl, heterocyclyl, alkoxycarbonylalkyl, aralkoxycarbonylalkyl, heteroaralkyloxycarbonylalkyl or G¹G²N-, G¹G²NC(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. Alkyl, cycloalkyl, aryl, heteroaryl, heterocyclyl, alkoxycarbonylalkyl, aralkoxycarbonylalkyl, heteroaralkyloxycarbonylalkyl or G¹G²N-, G¹G²NC(=O)-alkyl, annelated arylheterocyclenyl, annelated arylheterocyclyl are the preferred "carbamoyl substituents". The meanings of "carbamoyl substituents" are defined in this section.
**"Carboxylic substituent"** means a substituent attached to O-atom of carboxyl group the meaning of which is defined in this section. The substituent represents alkyl, cycloalkyl, aryl, heteroaryl, heterocyclyl, alkoxycarbonylalkyl, aralkoxycarbonylalkyl, heteroaralkyloxycarbonylalkyl or G¹G²N-, G¹G²NC(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. The preferred "carboxylic substituents" are alkyl, cycloalkyl, aryl, heteroaryl, heterocyclyl, alkoxycarbonylalkyl, aralkoxycarbonylalkyl, heteroaralkyloxycarbonylalkyl or G¹G²N- alkyl, G¹G²NC(=O)-alkyl, annelated arylheterocyclenyl, annelated arylheterocyclyl.
**"Nucleophilic substituent"** is a chemical radical attached to the scaffold as a result of a reaction with a nucleophilic reagent, for example, one selected from a group of primary or secondary amines, alcohols, phenols, mercaptans and thiophenols.
**"Cyclic system substituent"** means a substituent attached to an aromatic or nonaromatic cyclic system selected from hydrogen, alkyl, alkenyl, alkynyl, aryl, heteroaryl, aralkyl, heteroaralkyl, hydroxy, hydroxyalkyl, alkoxy, aryloxy, acyl, aroyl, halogen, nitro, cyano, carboxy, alkoxycarbonyl, aryloxycarbonyl, aralkoxycarbonyl, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, cycloalkyl, cycloalkenyl, heterocyclyl, heterocyclenyl, amidino, G¹G²N-, G¹G²N-alkyl, G¹G²NC(=O)- or G¹G²NSO₂-, where G¹ and G² independently of each other represent hydrogen, optionally substituted alkyl, optionally substituted aryl, optionally substituted aralkyl, optionally substituted heteroaralkyl or G¹G²N-substituent in which one of G¹ and G² could be acyl or aroyl, the meaning of the second substituent is defined above, or "cyclic system substituent" is G¹G²NC(=O)- or G¹G²NSO₂-, where G¹ and G² together with the N-atom they are attached to via G¹ and G² form 4-7-membered heterocyclyl or hetrocyclenyl. The preferred "cyclic system substituents" are alkoxycarbonyl, alkoxy, halogen, aralkoxy, alkyl, hydroxy, aryloxy, nitro, cyano, alkylsulfonyl, heteroaryl or G¹G²N-. If the cyclic system is saturated of partly saturated "cyclic system substituent" may have the meanings: methylene (CH₂=), oxo (O=) or thioxo (S=).
**"Electrophilic substituent"** means a chemical radical attached to the scaffold as a result of a reaction with an electrophilic reagent, for example, one selected from a group of organic acids or their derivatives (anhydrides, imidazolides, acid halides), organic sulfonic acid esters or chlorides, organic haloformates, organic isocyanates and organic isothiocyanates.
**"Protective group" (PG)** means a chemical radical attached to a scaffold or synthetic intermediate for temporary protection of amino group in multifunctional compounds, including, but not limited to: amide substituent, such as formyl, optionally substituted acetyl (for example, trichloroacetyl, trifluoroacetyl, 3-phenylpropionyl and others), optionally substituted benzoyl and others; carbamate substituent, such as optionally substituted C₁-C₇-alkoxycarbonyl, for example, methyloxycarbonyl, ethyloxycarbonyl, tert-butyloxycarbonyl, 9-fluorenylmethyloxycarbonyl (Fmoc) and others; optionally substituted C₁-C₇-alkyl substituent, for example, tert-butyl, benzyl, 2,4-dimethoxybenzyl, 9-phenylfluorenyl and others; sulfonyl substituent, for example, benzenesulfonyl, p-toluenesulfonyl and others. More specifically "Protective groups" are described in the book: Protective groups in organic synthesis, Third Edition, Green, T.W. and Wuts, P.G.M. 1999, p.494-653. Jon Wiley & Sons, Inc., New York, Chichester, Weinheim, Brisbane, Toronto, Singapore.
"**Protected primary or secondary amine"** means a group of the general formula G¹G²N-, where G¹ represents a protective group PG and G² is hydrogen, alkyl, alkenyl, aralkyl, aryl, annelated arylcycloalkenyl, annelated arylcycloalkyl, annelated arylheterocyclenyl, annelated arylheterocyclyl, cycloalkyl, cycloalkenyl, heteroaralkyl, heteroaryl, annelated heteroarylcycloalkenyl, annelated heteroarylcycloalkyl, annelated heteroarylheterocyclenyl, annelated heteroarylheterocyclyl, heterocyclenyl or heterocyclyl.
**"Inert substituent"** ("non-interfering substituent") means a low- or non-reactive radical, including, but not limited to: C₁-C₇ alkyl, C₂-C₇ alkenyl, C₂-C₇ alkynyl, C₁-C₇ alkoxy, C₇-C₁₂ aralkyl, substituted by inert substituents aralkyl, C₇-C₁₂ heterocyclylalkyl, substituted by inert substituents heterocyclylalkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₂-C₁₀ alkylsulfinyl, C₂-C₁₀ alkylsulfonyl, (CH₂)ₘ-O-(C₁-C₇ alkyl), -(CH₂)ₘ-N(C₁-C₇ alkyl)ₙ, aryl; aryl substituted by halogen or inert substituent; alkoxy group substituted by inert substituent; fluoroalkyl, aryloxyalkyl, heterocyclyl; heterocyclyl substituted by inert substituents and nitroalkyl; where m and n are varied from 1 to 7. The preferred inert substituents are C₁-C₇ alkyl, C₂-C₇ alkenyl, C₂-C₇ alkynyl, C₁-C₇ alkoxy, C₇-C₁₂ aralkyl, C₇-C₁₂ alkaryl, C₃-C₁₀ cycloalkyl, C₃-C₁₀ cycloalkenyl, C₁-C₇ alkyl substituted by inert substituents, phenyl; phenyl substituted by inert substituents; (CH₂)ₘ-O-(C₁-C₇ alkyl), -(CH₂)ₘ-N(C₁-C₇ alkyl)ₙ, aryl; aryl substituted by inert substituents, heterocyclyl and heterocyclyl substituted by inert substituents.
**"Carbamoyl"** means G¹G²NC(=O)- group. Carbamoyl may have one or more the same or different "carbamoyl substituents" G¹ and G² including alkenyl, alkyl, aryl, heteroaryl, heterocyclyl, the meanings of which are defined in this section.
**"Carbamoylazaheterocycle"** means azaheterocycle with at least one carbamoyl group as a "cyclic system substituent". The meanings of "azaheterocycle", "cyclic system substituent", and "carbamoyl group" are defined in this section.
**"Carboxy"** means HOC(=O)-(carboxyl) group.
**"Carboxyalkyl"** means HOC(=O)-alkyl-group wherein the meaning of alkyl is defined in this section.
**"Carbocycle"** means mono- or polycyclic system consisting of carbon atoms only. Carbocycles could be both aromatic and alicyclic. Alicyclic polycycles may have one or more common atoms. One common atom leads to spiro-carbocycles (for example, spiro[2,2]pentane); two - various condensed system (for example, decaline); three common atoms - to bridged systems (for example, bicycle[3,3,1]nonane); the greater number of common atoms leads to various polyhedron systems (for example, adamantane). Alicycles could be "saturated", for instance as cyclohexane, or "partly saturated" as tetraline.
**"Combinatorial library"** means a collection of compounds produced by parallel synthesis and intended for searching a hit or leader compound, and for optimization of physiological activity of the hit or leader as well, moreover each compound of the library corresponds to the common scaffold, in this way the library is a collection of related homologues or analogues.
**"Methylene radical"** means -CH₂-group with one or two "alkyl substituents" of the same or different structure, the meanings of which are defined in this section.
**"Nonaromatic cycle"**(saturated or partly saturated cycle) means nonaromatic mono- or polycyclic system formally generated as a result of complete or partial hydrogenization of unsaturated -C=C- or -C=N- bonds. Nonaromatic cycle may have one or more "cyclic system substituents" and could be annelated to aromatic, heteroaromatic or heterocyclic systems. Cyclohexane and piperidine are examples of nonaromatic cycles; cyclohexene and piperideine - are partly saturated cycles.
**"Optionally aromatic cycle"** means a cycle which could be both aromatic and nonaromatic, the meanings of which are defined in this section.
**"Optionally substituted radical"** means a radical without or with one or more substituents.
**"Optionally annelated (condensed) cycle"** means a condensed or noncondensed cycle, the meanings of which are defined in this section.
**"Lower alkyl"** means a straight or branched alkyl radical with 1 - 4 carbon atoms.
**"Leader compound"** (leader) means a compound of outstanding (maximum) physiological activity associated with a concrete biotarget related to a definite (or several) pathology or disease.
**"Hit compound"** (hit) means a compound demonstrated the desired physiological activity during the primary screening process.
**"Sulfamoyl group"** means G¹G²NSO₂-group substituted or not by "amino group substituens" G¹ and G², the meanings of which are defined in this section.
**"Sulfonyl"** means G³-SO₂-group wherein G³ could be selected from alkyl, cycloalkyl, aryl, heteroaryl, heterocyclyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, the meanings of which are defined in this section.
**"Template"** means the common structural fragment of the group of the compounds or compounds forming the combinatorial library.
**"Thiocarbamoyl"** means G¹G²NC(=S)-group. Thiocarbamoyl may have one or more "amino group substituents" G¹ and G², the meanings of which are defined in this section, for example, alkyl, alkenyl, aryl, heteroaryl and heterocyclyl the meanings of which are defined in this section.
**"Cycloalkyl"** means a radical derived from nonaromatic mono- or polycyclic system with 3 - 10 carbon atoms. Cycloalkyl may have one or more "cyclic system substituents" of the same or different structure. The representatives of cycloalkyl groups are cyclopropyl, cyclobutyl, cyclopentyl, cyclohexyl, decalinyl, norbornyl, adamant-1-yl and others. Cycloalkyl could be annelated with aromatic cycle or heterocycle. The preferred "cyclic system substituents" are alkyl, aralkoxy, hydroxy or G¹G²N- the meanings of which are defined in this section. **"Cycloalkylcarbonyl"** means cycloalkyl-C(=O)-group wherein the meaning of cycloalkyl is defined in this section. The representatives of cycloalkylcarbonyl groups are cyclopropylcarbonyl or cyclohexylcarbonyl.
**"Cycloalkoxy"** means cycloalkyl-O-group wherein the meaning cycloalkyl is defined in this section.
**"Pharmaceutical composition"** means a composition containing at least one biologically active compound (substance) and at least one of the components selected from a group consisting of pharmaceutically acceptable and pharmacologically compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and the way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and the mixtures of thereof as well. Protection against the effect of microorganisms can be provided by various antibacterial and antifungal agents, such as, for example, parabens, chlorobutanole, sorbic acid, and similar compounds. A composition may also contain isotonic agents, such as, for example, sugar, sodium chloride, and similar compounds. A prolonged effect of the composition may be achieved by agents slowing down absorption of the active ingredient, for example, aluminum monostearate and gelatine. Examples of suitable carriers, solvents, diluents and delivery agents include water, ethanol, polyalcohols and their mixtures, natural oils (such as olive oil) and for injection-grade organic esters (such as ethyl oleate). Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and high molecular weight polyethylene glycol. A pharmaceutical composition for peroral, sublingval, transdermal, intramuscular, intravenous, subcutaneous, local or rectal administration of the active ingredient, alone or in combination with another active compound may be administered to humans and animals in a standard administration form, or in a mixture with traditional pharmaceutical carriers. Suitable standard administration forms include peroral forms such as tablets, gelatin capsules, pills, powders, granules, chewing-gums and peroral solutions or suspensions; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. The salts could be prepared ***in situ*** in the processes of synthesis, isolation or purification of compounds or they could be prepared specially. In particular, base's salts could be prepared starting from purified base of the disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of the properties of such salts is given in: Berge S.M., et al., "Pharmaceutical Salts" J.Pharm.Sci., 1977, 66: 1-19). Salts of the disclosed acids may be also prepared by the reaction of purified acids specifically with a suitable base; moreover, metal salts and amine salts may be synthesized too. Metal salts are salts of sodium, potassium, calcium, barium, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of the sufficient basicity to produce a stable salt and suitable for use for medical purposes (in particular they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.
**"Focuced library"** means a combinatorial library or a collection of several combinatorial libraries or a collection of libraries and compounds arranged in a special way for the purpose of increased probability of finding hit-compounds and leader-compounds or with the purpose of intensification of optimization thereof. As a rule, the design of focused libraries is associated with directed search for effectors (inhibitors, activators, agonists, antagonists, and so on) of definite biotargets (enzymes, receptors, ion channels, and so on).
**"Fragment"** (scaffold) means a molecular frame typical for the group of compounds or compounds belonging to the "combinatorial library". **"1,2-Ethylene radical"** means -CH₂-CH₂-group containing one or more "alkyl substituents" of the same or different structure, the meanings of which are defined in this section.

The purpose of the present invention is the development of novel ligands of 5-HT₆ receptor.

The purpose in view is achieved by utilization of annelated azaheterocyclic compounds of the general formula **1** or racemates, or optical isomers, or geometrical isomers, or pharmaceutically acceptable salts and/or hydrates thereof as ligands of 5-HT₆ receptor, 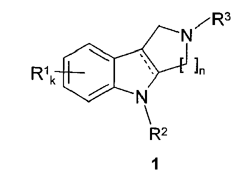 wherein:
R² and R³ independently of each other represent amino group substituent selected from hydrogen; substituted carbonyl; substituted aminocarbonyl; substituted aminothiocarbonyl; substituted sulfonyl; C₁-C₅-alkyl optionally substituted with C₆-C₁₀-aryl, heterocyclyl, C₆-C₁₀-arylaminocarbonyl, C₆-C₁₀-arylaminothiocarbonyl, C₅-C₁₀-azaheteroaryl, optionally substituted carboxyl, CN-group; optionally substituted aryl;
R¹ₖ represents from 1 to 3 substituents of cyclic system, independent of each other and selected from hydrogen, optionally substituted C₁-C₅-alkyl, C₁-C₅-alkoxy, C₁-C₅-alkenyl, C₁-C₅-alkynyl, halogen, trifluoromethyl, CN-group, carboxyl, optionally substituted aryl, optionally substituted heterocyclyl, substituted sulfonyl, optionally substituted carboxyl; the solid line accompanied by the dotted line, i.e. (---) represents a single or a double bond; n=1,2 or 3.

According to the invention the preferable azaheterocyclic compounds as ligands of 5-HT₆ receptor are substituted pyrrolo[4,3-b]indoles of the general formula **1.1** or racemates, or optical isomers, or geometrical isomers, or pharmaceutically acceptable salts and/or hydrates thereof, 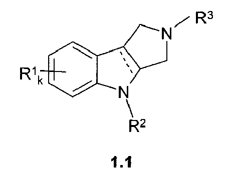 wherein:
R¹ₖ, R², R³ and the solid line accompanied by the dotted line, i.e. (---) are all as defined above.

According to the invention the preferable azaheterocyclic compounds as ligands of 5-HT₆ receptor are substituted 1,2,3,4-tetrahydropyrrolo[4,3-b]indoles of the general formula **1.2** or racemates, or optical isomers, or geometrical isomers, or pharmaceutically acceptable salts and/or hydrates thereof, 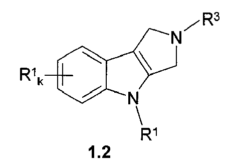 wherein:
R¹ₖ, R² and R³ are all as defined above.

According to the invention the preferable azaheterocyclic compounds as ligands of 5-HT₆ receptor are substituted *cis*-1,2,3,3a,4,8b-hexahydropyrrolo[4,3-b]indoles of the general formula **1.3** or racemates, or optical isomers, or geometrical isomers, or pharmaceutically acceptable salts and/or hydrates thereof, 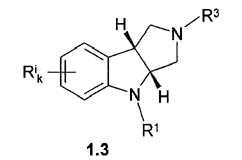 wherein:
R¹ₖ, R² and R³ are all as defined above.

According to the invention the preferable azaheterocyclic compounds as ligands of 5-HT₆ receptor are substituted γ-carbolines of the general formula **1.4** or racemates, or optical isomers, or geometrical isomers, or pharmaceutically acceptable salts and/or hydrates thereof, 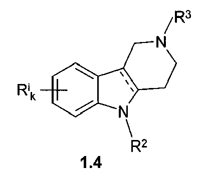 Wherein:
R¹ₖ, R², R³ and the solid line accompanied by the dotted line (---) are all as defined above.

According to the invention the preferable azaheterocyclic compounds as ligands of 5-HT₆ receptor are substituted 2,3,4,5-tetrahydro-γ-carbolines of the general formula **1.5** or racemates, or optical isomers, or geometrical isomers, or pharmaceutically acceptable salts and/or hydrates thereof, 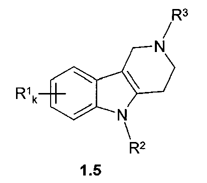 Wherein:
R¹ₖ, R² and R³ are all as defined above.

According to the invention the preferable azaheterocyclic compounds as ligands of 5-HT₆ receptor are substituted 2,3,4,4a,5,9b-hexahydro-γ-carbolines of the general formula **1.6** or racemates, or optical isomers, or geometrical isomers, or pharmaceutically acceptable salts and/or hydrates thereof, 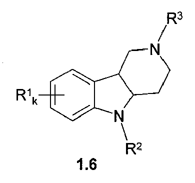 Wherein:
R¹ₖ, R² and R³ are all as defined above.

According to the invention the preferable azaheterocyclic compounds as ligands of 5-HT₆ receptor are substituted *cis*-2,3,4,4a,5,9b-hexahydro-γ-carbolines of the general formula **1.7** or racemates, or optical isomers, or geometrical isomers, or pharmaceutically acceptable salts and/or hydrates thereof, 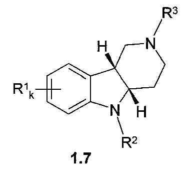 Wherein:
R¹ₖ, R² and R³ are all as defined above.

According to the invention the preferable azaheterocyclic compounds as ligands of 5-HT₆ receptor are substituted *trans*-2,3,4,4a,5,9b-hexahydro-γ-carbolines of the general formula **1.8** racemates, or optical isomers, or geometrical isomers, or pharmaceutically acceptable salts and/or hydrates thereof, 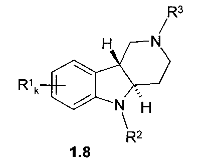 Wherein:
R¹ₖ, R² and R³ are all as defined above.

According to the invention the preferable azaheterocyclic compounds as ligands of 5-HT₆ receptor are substituted azepino[4,3-b]indoles of the general formula **1.9** or racemates, or optical isomers, or geometrical isomers, or pharmaceutically acceptable salts and/or hydrates thereof, 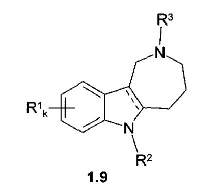 Wherein:
R¹ₖ, R², R³ and the solid line accompanied by the dotted line (---) are all as defined above.

According to the invention the preferable azaheterocyclic compounds as ligands of 5-HT₆ receptor are substituted 1,2,3,4,5,6-hexahydroazepino[4,3-b]indoles of the general formula **1.10** or racemates, or optical isomers, or geometrical isomers, or pharmaceutically acceptable salts and/or hydrates thereof, 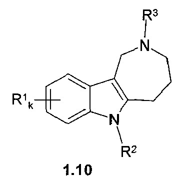 Wherein:
R¹ₖ, R² and R³ are all as defined above.

According to the invention the preferable azaheterocyclic compounds as ligands of 5-HT₆ receptor are substituted 1,2,3,4,5,5a,6,10b-octahydroazepino[4,3-b]indoles of the general formula **1.11** or racemates, or optical isomers, or geometrical isomers, or pharmaceutically acceptable salts and/or hydrates thereof, 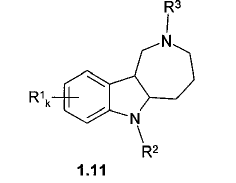 Wherein:
R¹ₖ, R² and R³ are all as defined above.

According to the invention the preferable azaheterocyclic compounds as ligands of 5-HT₆ receptor are substituted *cis*-1,2,3,4,5,5a,6,10b-octahydroazepino[4,3-b]indoles of the general formula **1.12** or racemates, or optical isomers, or geometrical isomers, or pharmaceutically acceptable salts and/or hydrates thereof, 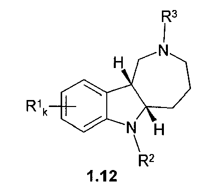 Wherein:
R¹ₖ, R² and R³ are all as defined above.

According to the invention the preferable azaheterocyclic compounds as ligands of 5-HT₆ receptor are substituted *trans*-1,2,3,4,5,5a,6,10b-octahydroazepino[4,3-b]indoles of the general formula **1.13** or racemates, or optical isomers, or geometrical isomers, or pharmaceutically acceptable salts and/or hydrates thereof, 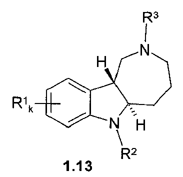 Wherein:
R¹ₖ, R² and R³ are all as defined above.

The purpose of the present invention is the development of pharmaceutical composition for prophylaxis and treatment of various diseases of CNS of warm-blooded animals and humans pathogenesis of which is associated with 5-HT₆ receptors.

The purpose in view is achieved by a pharmaceutical composition for preparation of medicaments for treatment and prophylaxis of diseases and conditions of central nervous system (CNS) pathogenesis of which is associated with 5-HT6 receptors comprising as an active ingredient pharmaceutically effective amount of azaheterocyclic compound of the general formula **1** or racemate, or optical isomer, or geometrical isomer, or pharmaceutically acceptable salt and/or hydrate thereof, 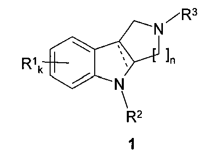 Wherein: R¹ₖ, R², R³, the solid line accompanied by the dotted line (---) and n are all as defined above.

The purpose of the present invention is also a method for preparation of pharmaceutical composition.

The purpose in view is achieved by mixing at least one azaheterocyclic compound of the general formula **1,** or racemate, or optical isomer, or geometrical isomer or pharmaceutically acceptable salt and/or hydrate thereof with inert filler and/or diluents.

Pharmaceutically acceptable fillers and/or diluents (excipients) mean diluents, auxiliary agents and/or carriers employing in the sphere of pharmaceutics. According to the invention azaheterocyclic compounds of the general formula **1** could be used in combination with other active ingredients provided that they do not give rise to undesirable effects, for example, allergic reactions.

According to the present invention a pharmaceutical composition can be used in clinical practice in various forms prepared by mixing of the compositions with traditional pharmaceutical carries, for example, peroral forms (such as, tablets, gelatinous capsules, pills, solutions or suspensions); forms for injections (such as, solutions or suspensions for injections, or a dry powder for injections which requires only addition of water for injections before usage; local forms (such as, ointments or solutions).

The carriers used in pharmaceutical compositions, according to the present invention, represent carriers which are useful in the sphere of pharmaceutics for preparation of the commonly used forms including: binding agents, greasing agents, disintegrators, solvents, diluents, stabilizers, suspending agents, colorless agents, taste flavors are used in peroral forms; antiseptic agents, solubilizers, stabilizers are used in the forms for injections; base materials, diluents, greasing agents, antiseptic agents are used in local forms. Pharmaceuticals could be introduced peroral or parenteral (for example, intravenously, subcutaneously, intraperitoneally or locally). If any medicament is not stable in stomach, it is possible to use the tablets covered with coating soluble in stomach or intestine.

The purpose of the present invention is also a medicament for treatment and prophylaxis of diseases and conditions of central nervous system (CNS) pathogenesis of which is associated with 5-HT₆ receptors.

The purpose in view is achieved by a medicament in the form of tablets, capsules or injections, placed in a pharmaceutically acceptable packing for treatment and prophylaxis of diseases and conditions of central nervous system (CNS) pathogenesis of which is associated with 5-HT₆ receptors comprising a pharmaceutical composition including at least one ligand of the general formula **1.**

Besides, at patients the clinical dose of pharmaceutical composition or medicament comprising as an active ingredient an azaheterocyclic compound of the general formula 1, may be corrected depending on: therapeutic efficiency and bio-accessibility of active ingredients in their organism, the rate of their exchange and removal from organism, and also the age, gender, and severity of the patient's symptoms. Thus, the daily intake for adults normally being 10~500 mg, preferably 50~300 mg. Accordingly, the above effective doses are to be taken into consideration while preparing a medicament of the present invention from the pharmaceutical composition in the form of dose units, each dose unit of the preparation containing 10~500 mg of the compound of the general formula **1,** preferably 50~300 mg. Following the instructions of a physician or pharmacist, the medicines may be taken several times over specified period of time (preferably, from one to six times).

### Best Embodiment of the invention

The invention is illustrated by the following figures.
Fig. 1. Concentration dependence of the interaction level of azaheterocyclic compounds of the general formula **1** with 5-HT₆ receptor: ■ - **1.5(20),** □ - **1.10(50),** ▼ - **1.10(51),** ○ - **1.10(53),** ◆ - **1.13(13).**

Azaheterocyclic compounds of the general formula 1 or racemates, or optical isomers, or geometrical isomers, or pharmaceutically acceptable salts and/or hydrates thereof could be prepared by the known methods [Welch, W.M., Harbert, C.A., Weissman, A. J. Med. Chem. 1980, 23, 704-707. N. Barbulescu, C. Bomaz, C. si Greff - Rev. Chim. (Bucuresti), 1971, v.22, p. 269. Lermontova N.N., Lukoyanov N.V., Serkova T.P., Lukoyanova E.A., Bachurin S.O. Dimebon improves learning in animals with experimental Alzheimer's disease. Bull Exp Biol Med. 2000, 129(6), 544-546. Zefirov, N.S.; Afanasiev, A.Z.; Afanasievf, S.V.; Bachurin, S.E.; Tkachenko, S.E,; Grigoriev, V.V.; Jurovskaya, M.A.; Chetverikov, V.P.; Bukatina, E.E,; Grigoriev, I.V. US 6187785, 2001**],** many of them are commercially available from the american firm ChemDiv, Inc (San Diego, CA: www.chemdiv.com).

Below the invention is described by means of specific examples, which illustrate but not limit the scope of the invention.

### Example 1. Methods for preparation of substituted 1,2,3,4-tetrahydropyrrolo[4,3-b]indoles of the general formula 1.2, cis-1,2,3,3a,4,8b-hexahydropyrrolo[4,3-b]indoles of the general formula 1.3.

**A.** Preparation of 1,2,3,4-tetrahydropyrrolo[4,3-b]indoles of the general formula **1.2.** The compounds are prepared according to the method described for 2-carbethoxy-7-fluoro-1,2,3,4-tetrahydropyrrolo[4,3-b]indole **1.2(3)** [Welch, W.M., Harbert, C.A., Weissman, A. J. Med. Chem. 1980, 23, 704-707], among them: 2-carbethoxy-1,2,3,4-tetrahydropyrrolo[4,3-b]indole **1.2(1),** LCMS: m/z 231 [M+H] and others, some of them are represented in Table 1.
**B.** Method for preparation of substituted *cis*-1,2,3,3a,4,8b-hexahydropyrrolo[4,3-b]indoles of the general formula **1.3.** 2,7 Mmol of corresponding substituted 1,2,3,4-tetrahydropyrrolo[4,3-b]indole **1.2** and 150 mg of PtO₂ in 20 ml of ethanol are stirred in hydrogen atmosphere for 12-48 hr at 20°C. After the completion of hydrogenation (LCMS control) the reaction mixture is filtered, evaporated *in vacuo,* and subjected to chromatography on silica gel impregnated with Et₃N. It gives *cis*-1,2,3,3a,4,8b-hexahydropyrrolo[4,3-b]indoles **1.3,** among them: *cis*-2-(pyridin-3-ylmethyl)-1,2,3,3a,4,8b-hexahydropyrrolo[4,3-b]indole **1.3(1),** LCMS: m/z 252 [M+H]; *cis*-7-methyl-2-(3-fluorobenzyl)-1,2,3,3a,4,8b-hexahydropyrrolo[4,3-b]indole **1.3(2),** LCMS: m/z 283 [M+H] and others, some of them are represented in Table 1.

### Table 1. Examples of 5-HT₆ receptor ligands among the substituted pyrrolo[4,3-b]indoles of the general formulas 1.2 and 1.3.

Substituted pyrrolo[4,3-b]indoles of the general formula **1.2** 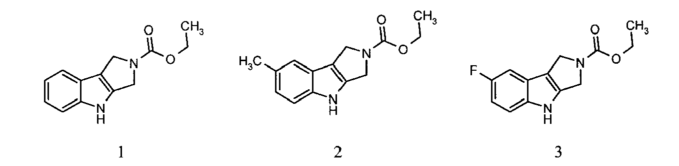 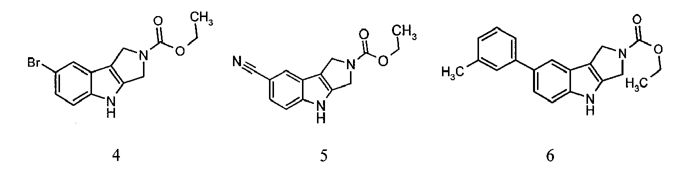 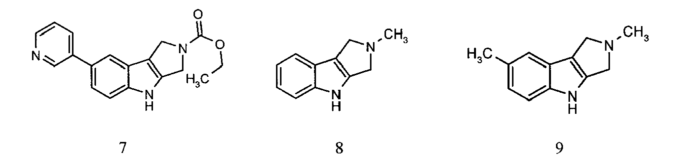 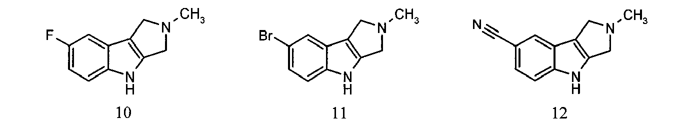 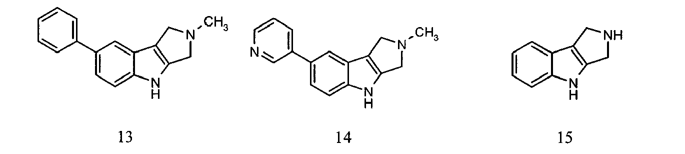 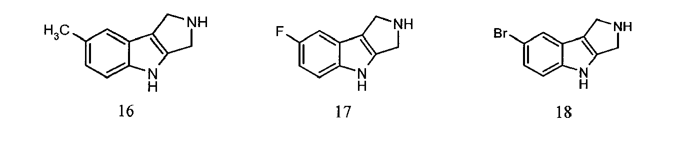 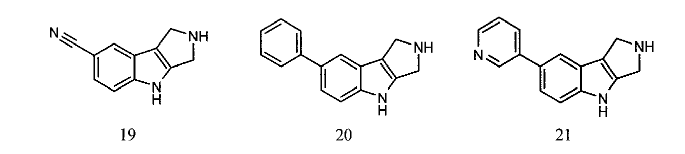 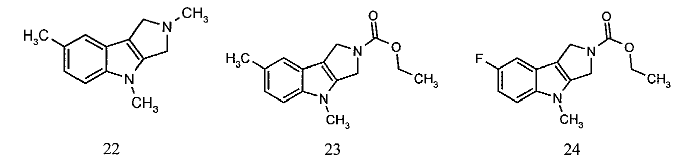 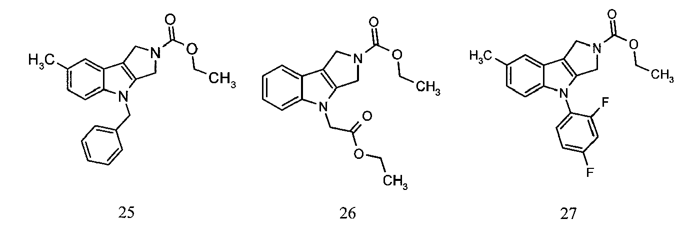 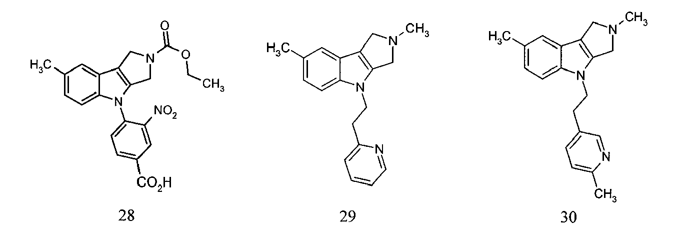 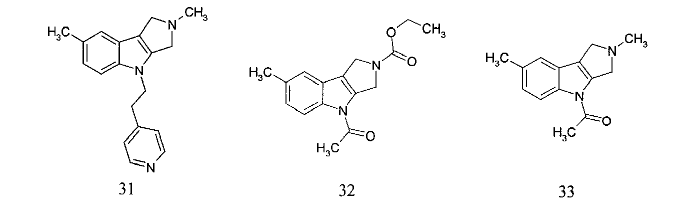 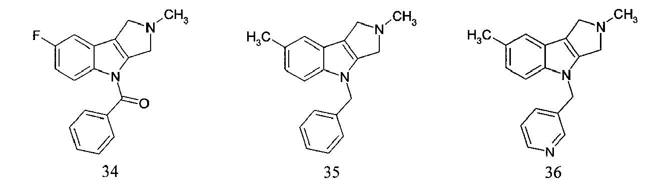 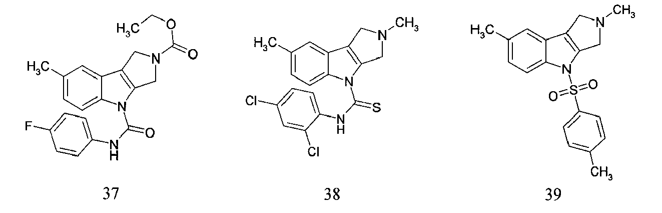 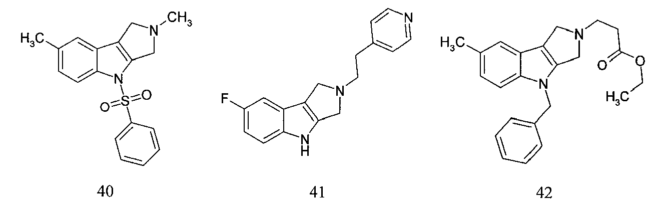 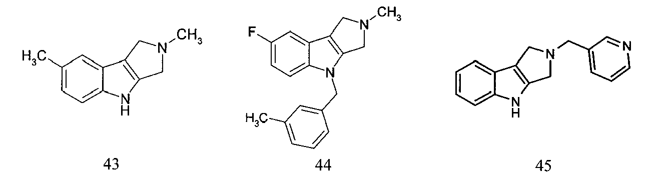 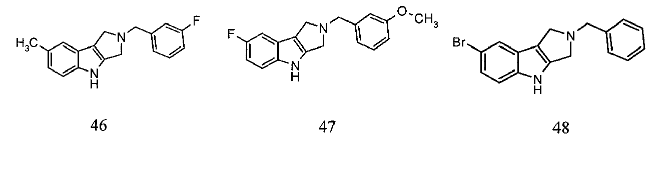 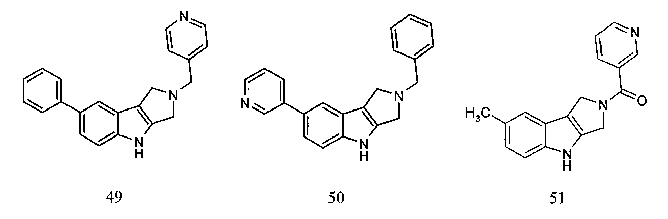 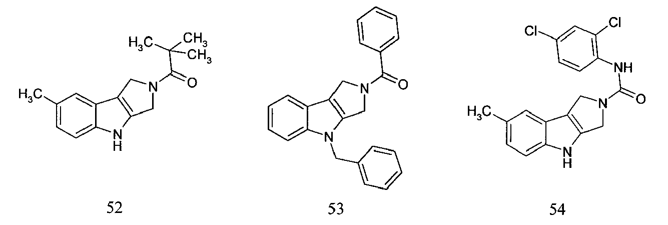 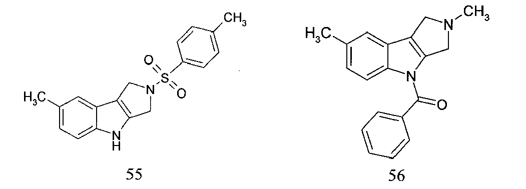 Substituted *cis*-1,2,3,3a,4,8b-hexahydropyrrolo[4,3-b]indoles of the general formula **1.3**

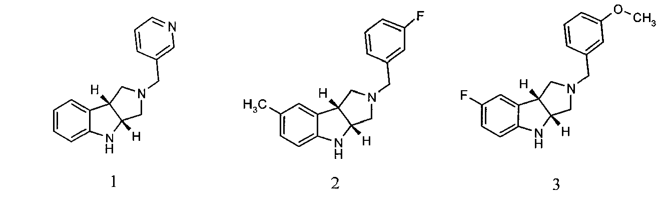

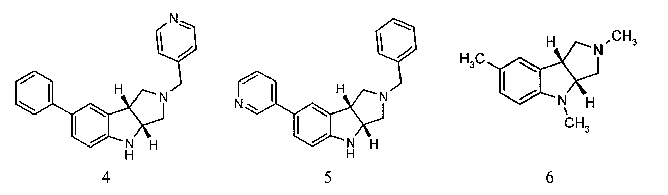

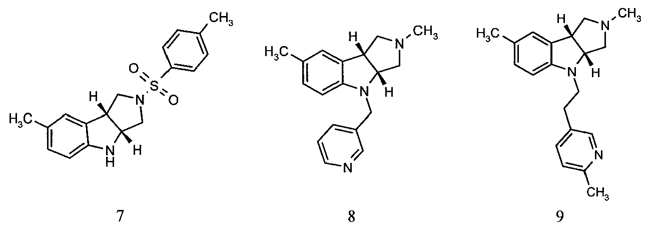

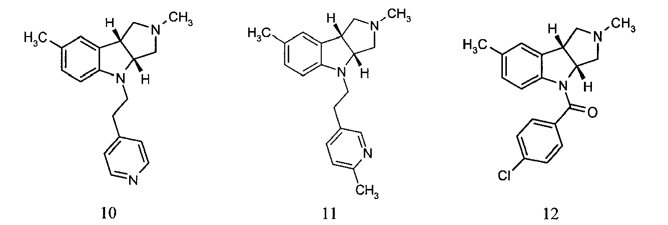

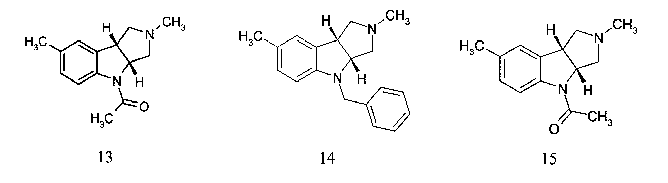

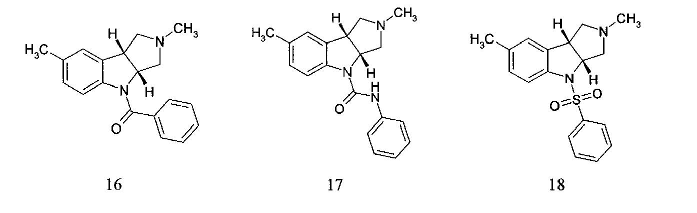

### Example 2. Method for preparation of substituted 2,3,4,5-tetrahydro-γ-carbolines of the general formula 1.5.

Synthesis of substituted 2,3,4,5-tetrahydro-γ-carbolines is carried out according to the known reaction of substituted phenylhydrazine (or its salts with mineral acids) with 1-substituted piperidin-4-ones [N. Barbulescu, C. Bornaz, C. si Greff - Rev. Chim. (Bucuresti), 1971, v.22, p. 269], among them 2,8-dimethyl-2,3,4,5-tetrahydro-γ-carboline 1.5(2), LCMS: m/z 201 [M+H] and others; some of them are represented in Table 2.

### Table 2. Examples of 5-HT₆ receptor ligands among substituted γ-carbolines of the general formulas 1.5, 1.6, 1.7.

2,3,4,5-Tetrahydro-γ-carbolines of the general formula **1.5**

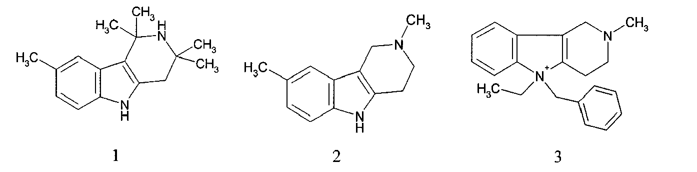

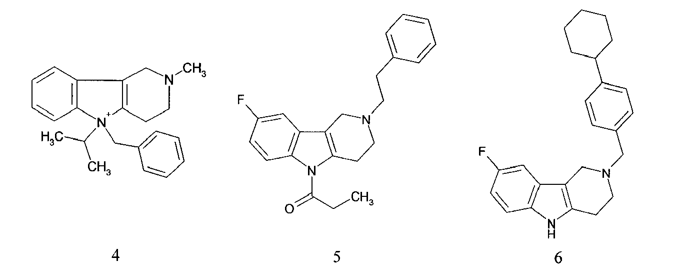

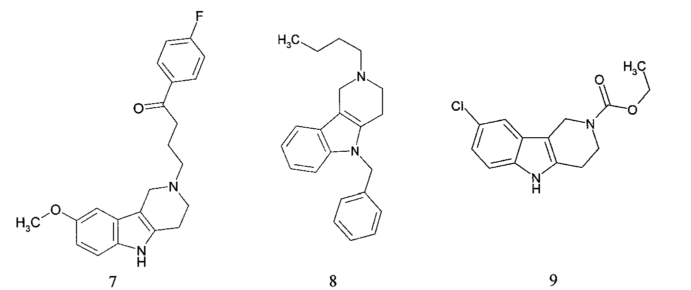

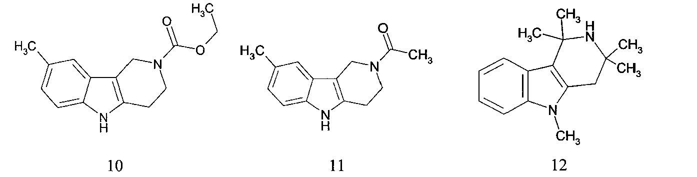

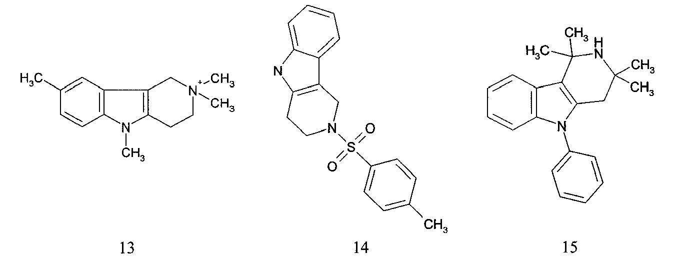

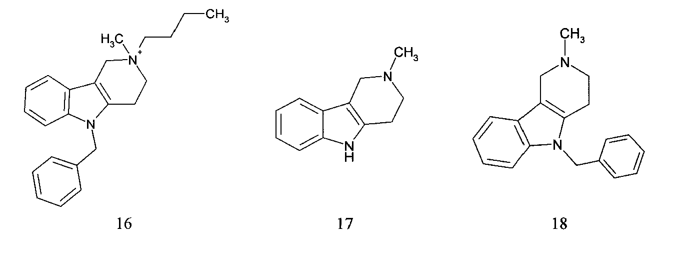

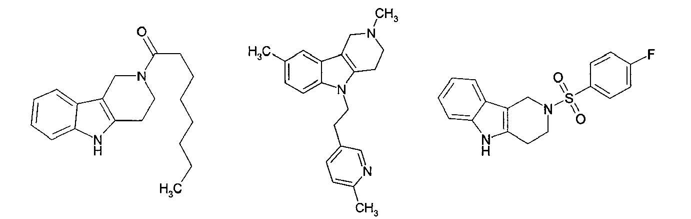

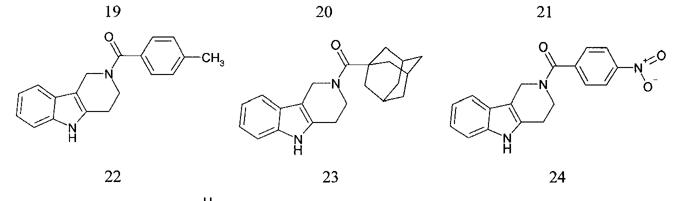

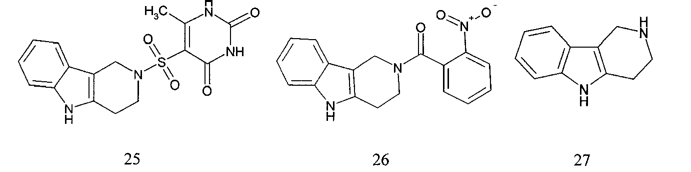

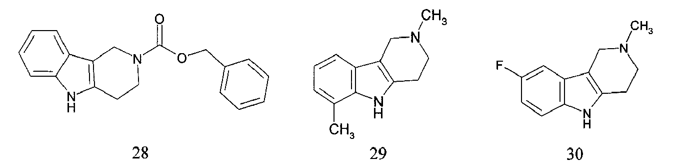

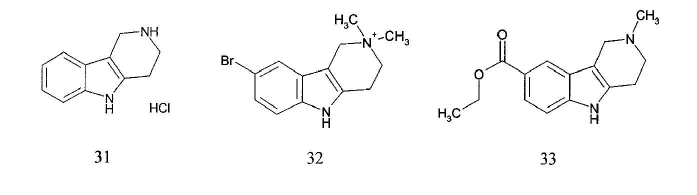

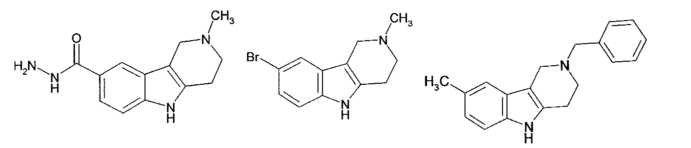

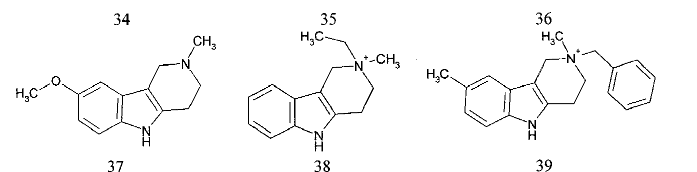

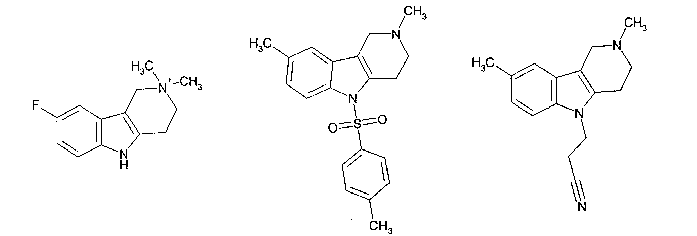

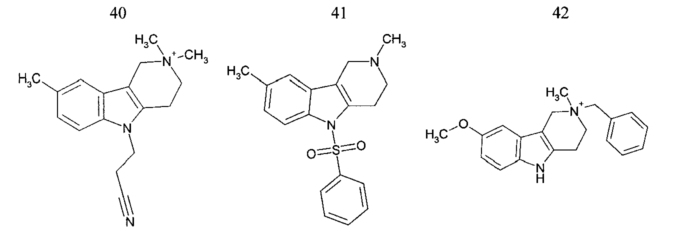

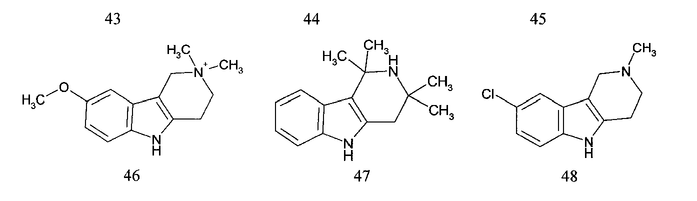

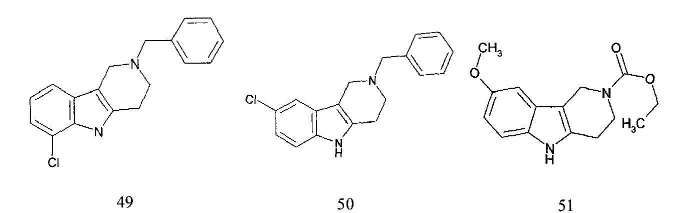

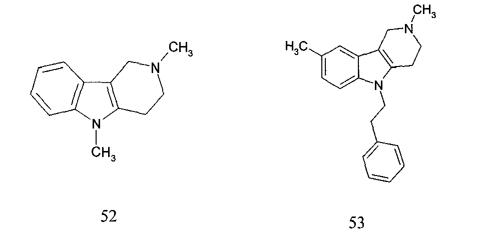

Substituted 2,3,4,4a,5,9b-hexahydro-γ-carbolines of the general formula **1.6**

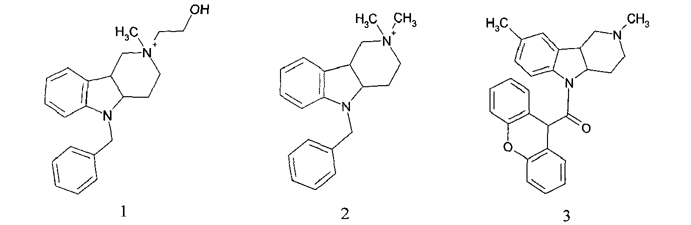

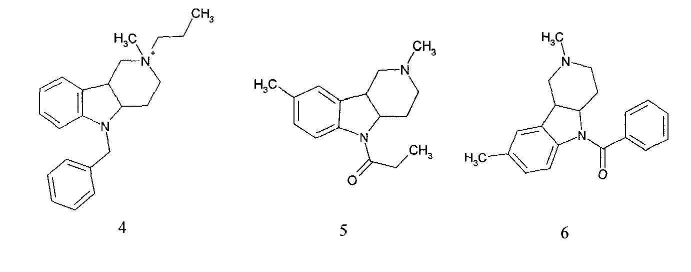

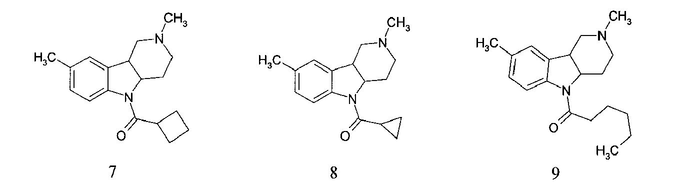

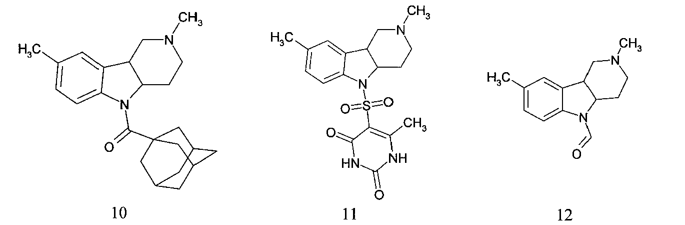

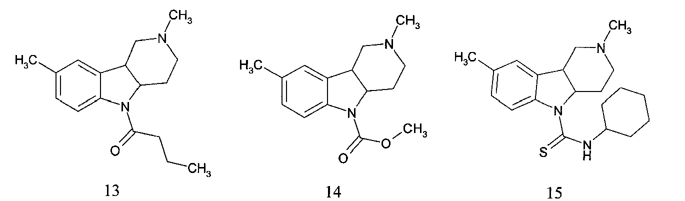

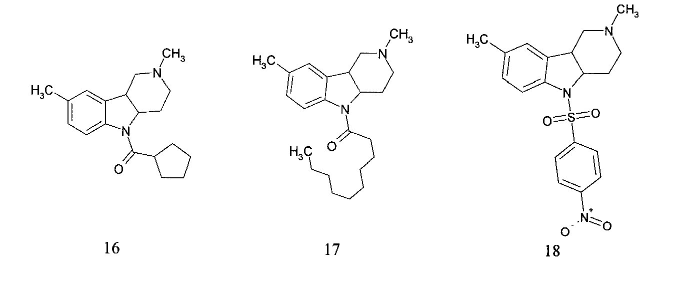

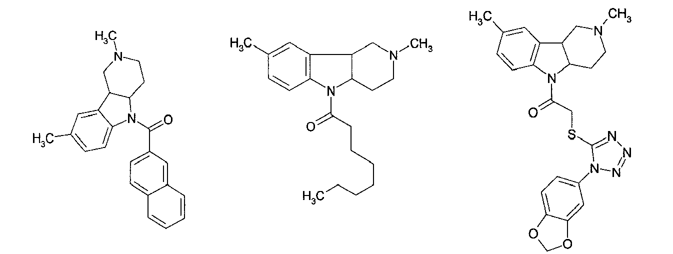

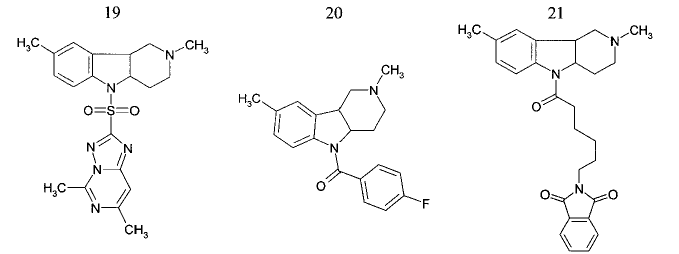

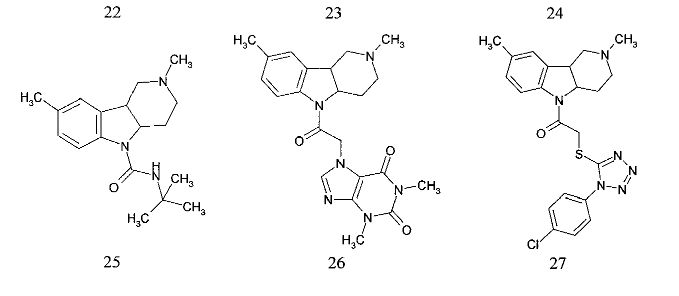

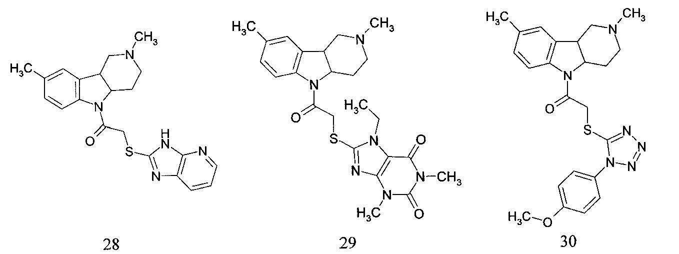

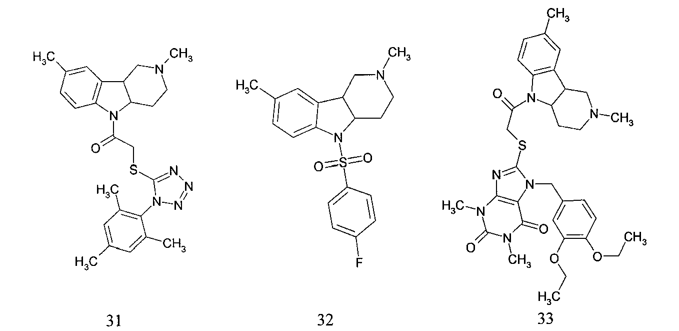

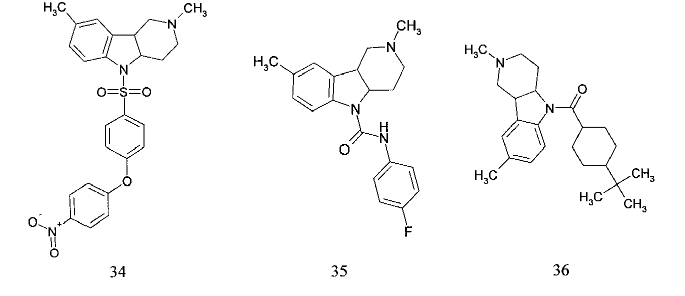

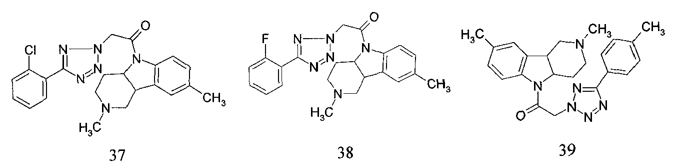

Substituted *cis*-2,3,4,4a,5,9a-hexahydro-γ-carbolines of the general formula **1.7**

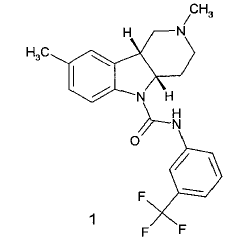

### Example 3. Methods for preparation of substituted 1,2,3,4,5,6-hexahydroazepino[4,3-b]indoles of the general formula 1.10, cis-1,2,3,4,5,5a,6,10b-octahydroazepino[4,3-b]indoles of the general formula 1.12, trans-1,2,3,4,5,5a,6,10b-octahydroazepino[4,3-b]indoles of the general formula 1.13.

**A.** Substituted 1,2,3,4,5,6-hexahydroazepino[4,3-b]indoles of the general formula **1.10** are prepared by the reduction of the corresponding 3,4,5,6-tetrahydro-2H-azepino[4,3-b]indol-1-ones with LiAlH₄ according to the method described for 1,2,3,4,5,6-hexahydroazepino[4,3-b]indole [Bascop, S.-I.; Laronze, J.-Y.; Sapi, J. Monatsh. Chemie 1999, 130, 1159-1166], among them: 9-methyl-1,2,3,4,5,6-hexahydroazepino[4,3-b]indole **1.10(1),** LCMS: m/z 201 [M+H]; 9-fluoro-1,2,3,4,5,6-hexahydroazepino[4,3-b]indole **1.10(2),** LCMS: m/z 205 [M+H]; 7,9-dimethyl-1,2,3,4,5,6-hexahydroazepino[4,3-b]indole **1.10(3),** LCMS: m/z 215 [M+H] and others, some of them are represented in Table 3.
**B.** Method for preparation of substiuted *cis*-1,2,3,4,5,5a,6,10b-octahydroazepino[4,3-b]indoles of the general formula **1.12.** Mixture of 2 mmol of the corresponding 1,2,3,4,5,6-hexahydroazepino[4,3-b]indole **1.10** and 500 mg (8 mmol) NaBH₃CN in 6 ml of acetic acid is stirred at room temperature for 12 hr (LCMS control). After the reaction is completed acetic acid is evaporated *in vacuo,* the residue is boiled for 10 min with 17,5% water solution of HCl. The transparent solution is cooled, alkalized with saturated water solution of NaOH, the product is extracted with a proper organic solvent. Extract is dried over Na₂SO₄ and evaporated *in vacuo.* The residue is subjected to chromatogrphy on silica gel impregnated with Et₃N (eluent: hexane-CHCl₃-Et₃N 4:4:2). It gives *cis*-1,2,3,4,5,5a,6,10b-octahydroazepino[4,3-b]indoles **1.12,** among them: *cis*-9-methyl-1,2,3,4,5,5a,6,10b-octahydroazepino[4,3-b]indole **1.12(2),** LCMS: m/z 203 [M+H], ¹H NMR (400 MHz, CDCl₃): 6.82-6.80 (m, 2H), 6.47-6.45 (m, 2H), 4.14-4.09 (m, 1H), 3.63-3.57 (m, 1H), 3.20-3.16 (m, 1H), 3.00-2.93 (m, 2H), 2.75-2.69 (m, 1H), 2.23 (s, 3H), 1.87-1.75 (m, 3H), 1.57-1.48 (m, 1H), ¹³C NMR (CDCl₃): δ= 20.66, 28.26, 33.01, 48.96, 51.13, 51.98, 62.41, 108.47, 124.67, 127.26, 127.91, 130.49, 148.35; *cis*-9-fluoro-1,2,3,4,5,5a,6,10b-octahydroazepino[4,3-b]indole **1.12(3),** LCMS: m/z 207 [M+H] and others, some of them are represented in Table 3.
**B.** Method for preparation of *trans*-1,2,3,4,5,5a,6,10b-octahydroazepino[4,3-b]indoles of the general formula **1.13.** 5 Mmol of 1,2,3,4,5,6-hexahydroazepino[4,3-b]indole **1.10** dissolved in 25 ml of THF is added to 10 ml of 1 M BH₃ solution in THF at stirring and 0-5°C. The reaction mixture is boiled for 1 hr in argon atmospere. After the reaction is completed (LCMS control) the solvent is evaporated *in vacuo,* 50 ml of 10% water solution of HCl is added to the residue and the resultant mixture is boiled for 30 min. After the hydrolysis is over (LCMS control) the reaction mixture is evaporated *in vacuo* to half of volume and alkalized with 10% water solution of KOH. The product is extracted with methylene chloride, the extract is dried over K₂CO₃, the solvent is evaporated *in vacuo,* and the residue is subjected to chromatography on silica gel impregnated with Et₃N. It gives *trans*-1,2,3,4,5,5a,6,10b-octahydroazepino[4,3-b]indoles **1.13,** yield 60-80%, among them: *trans*-2,9-dimethyl-1,2,3,4,5,5a,6,10b-octahydroazepino[4,3-b]indole **1.13(5),** LCMS: m/z 217 [M+H], ¹H NMR (400 MHz, DMSO-d6): 6.77 (s, 1H), 6.75 (d, 1H, J=7.69 Hz), 6.40 (d, 1H, J=7.69 Hz), 5.42 (br.s, 1H), 3.70 (m, 1H), 3.24 (m, 1H), 3.05 (m, 1H), 2.68 (m, 1H), 2.52 (m, 2H), 2.38 (s, 3H), 2.19 (s, 3H), 2.05 (m, 1H), 1.80-1.55 (m, 3H); *trans*-2-benzyl-9-methyl-1,2,3,4,5,5a,6,10b-octahydroazepino[4,3-b]indole **1.13(6),** LCMS: m/z 293 [M+H] and others, some of them are represented in Table 3.

### Table 3. Examples of 5-HT₆ receptor ligands among substituted azepino[4,3-b]indoles of the general formulas 1.10,1.12,1.13.

Substituted 1,2,3,4,5,6-hexahydroazepino[4,3-b]indoles **1.10** 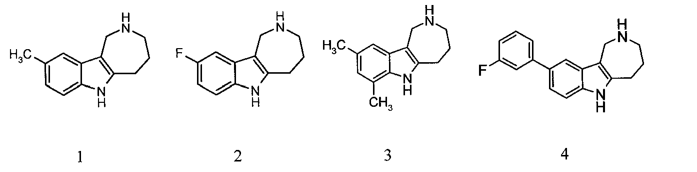 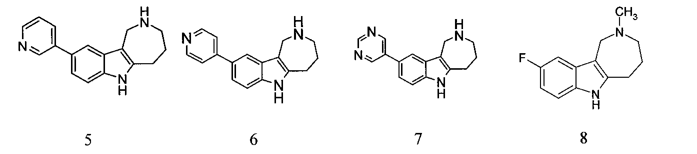 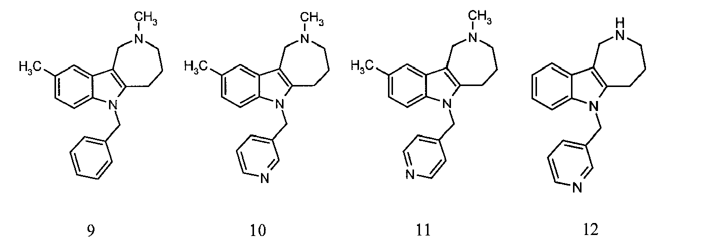 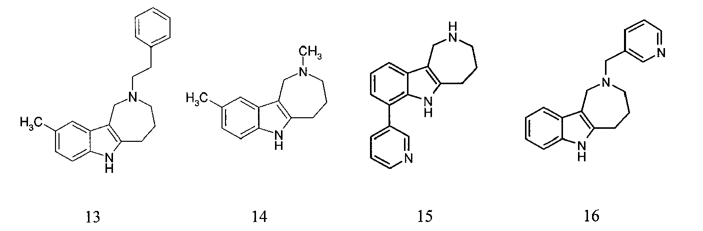 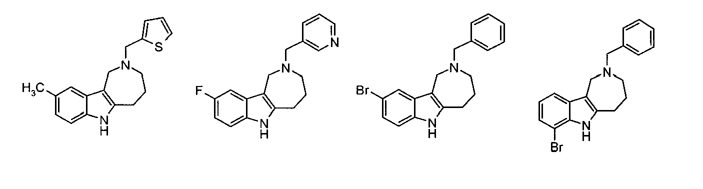 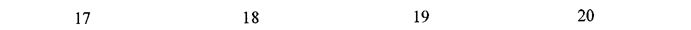 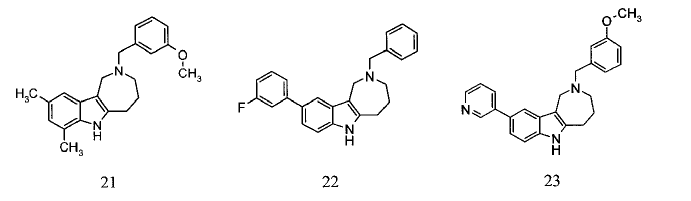 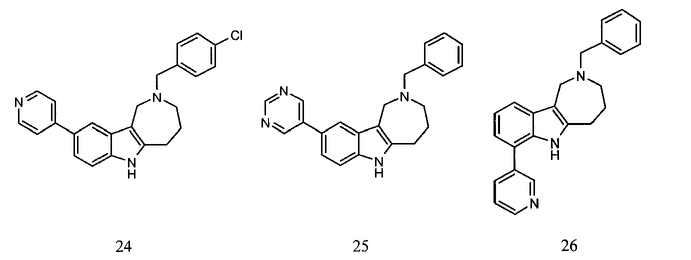 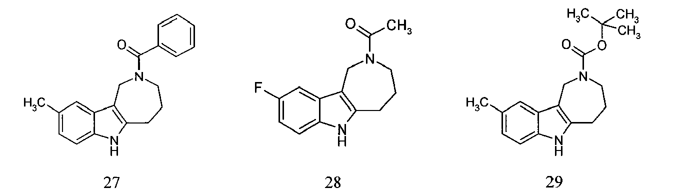 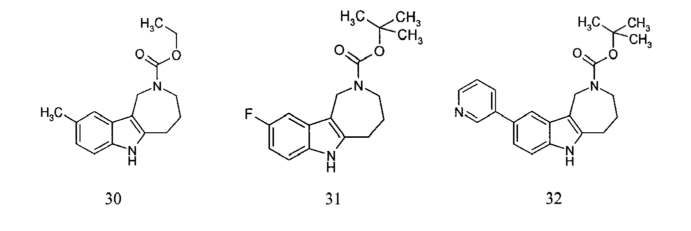 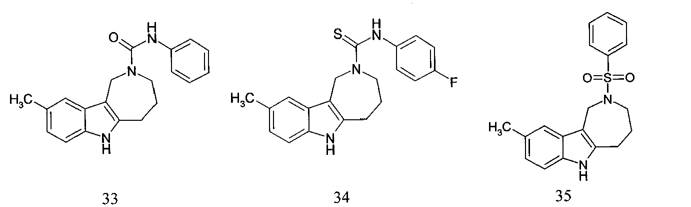 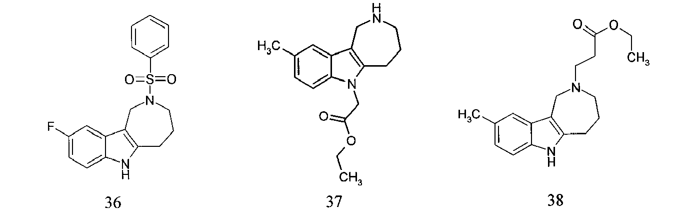 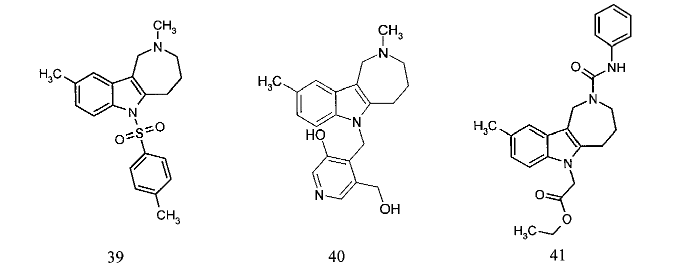 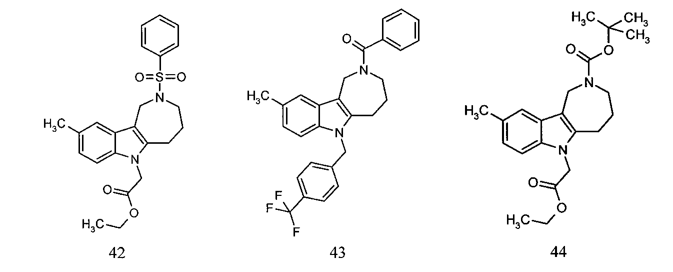 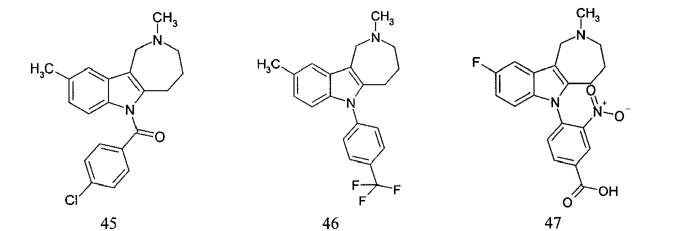 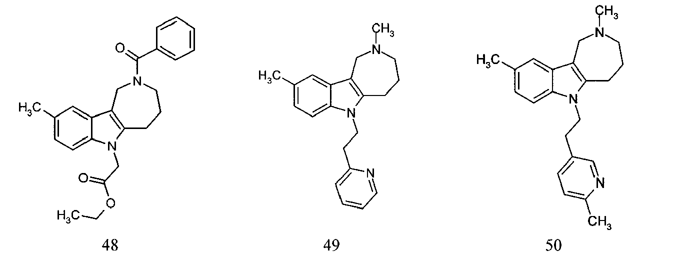 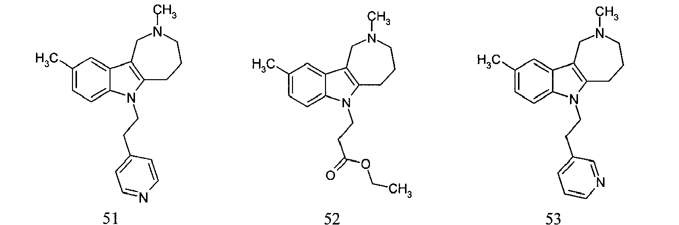 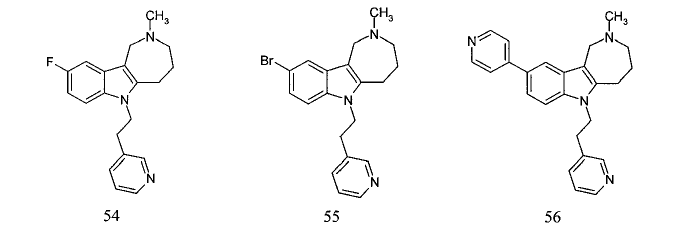 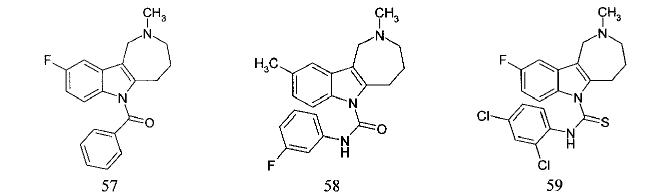 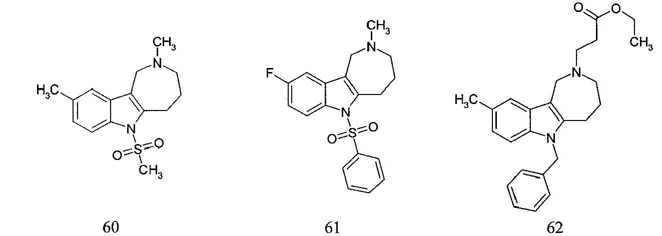 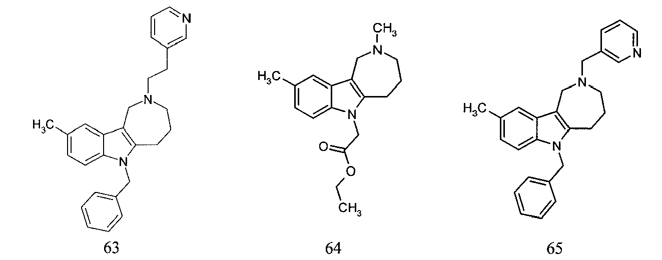 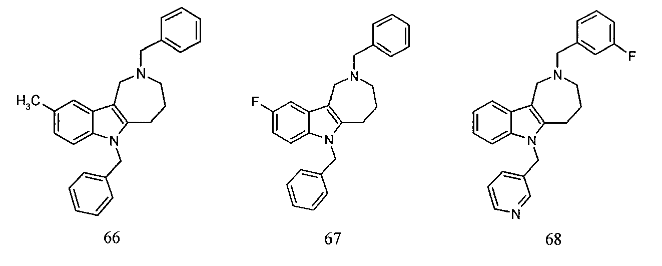 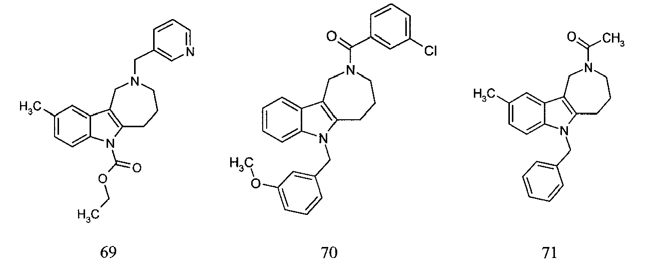 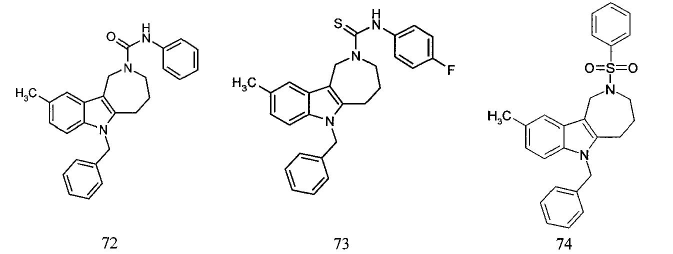 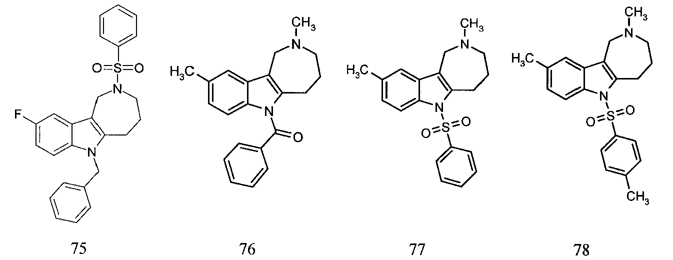 Substituted *cis*-1,2,3,4,5,5a,6,10b-octahydroazepino[4,3-b]indoles of the general formula **1.12**

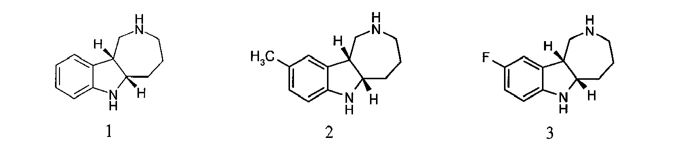

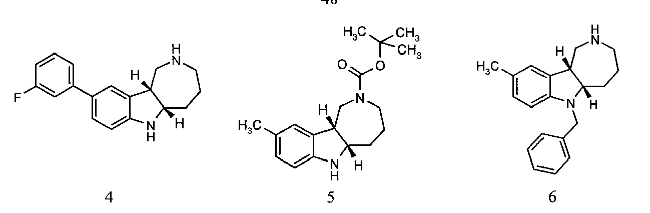

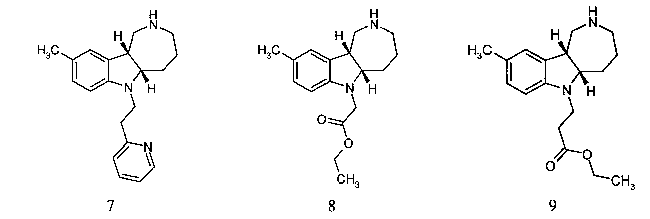

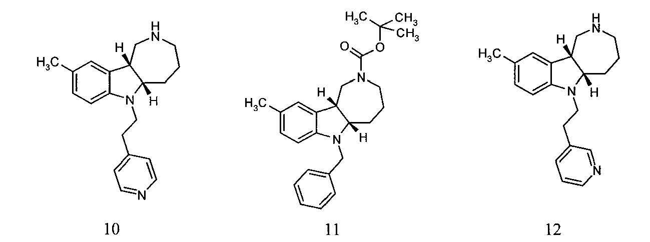

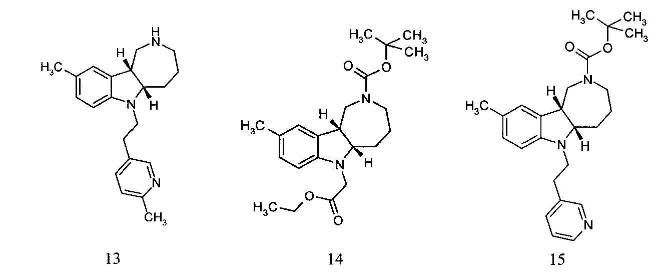

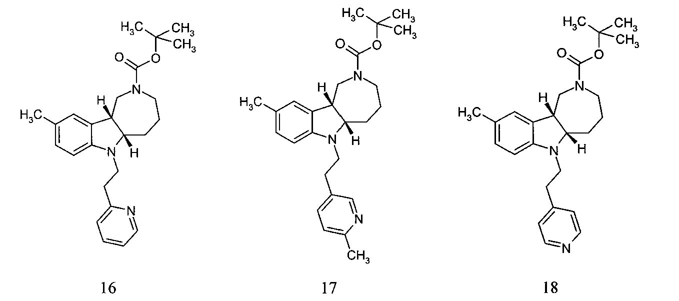

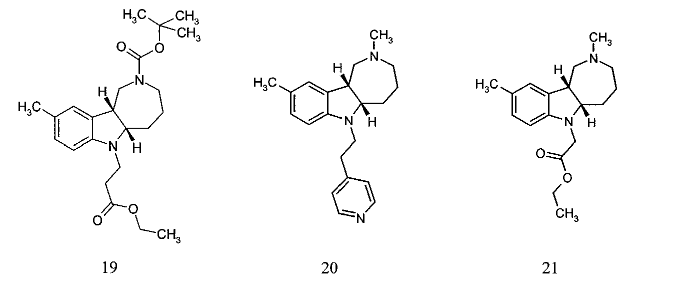

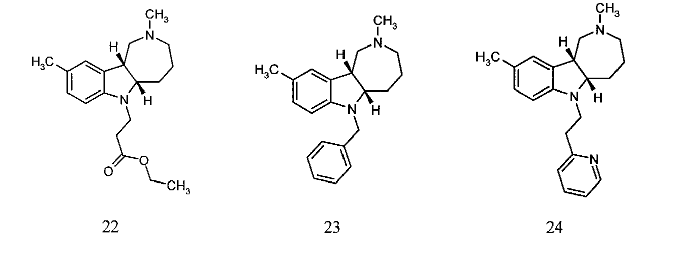

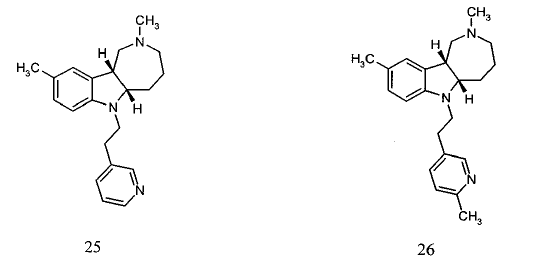

Substituted *trans*-1,2,3,4,5,5a,6,10b-octahydroazepino[4,3-b]indoles **1.13**

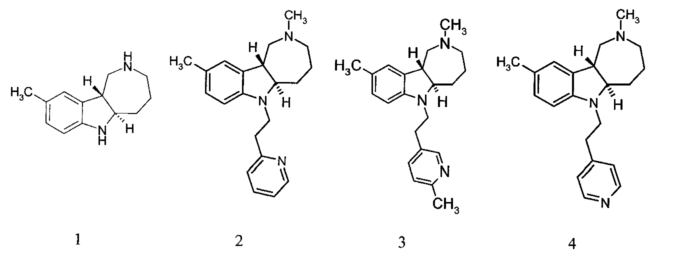

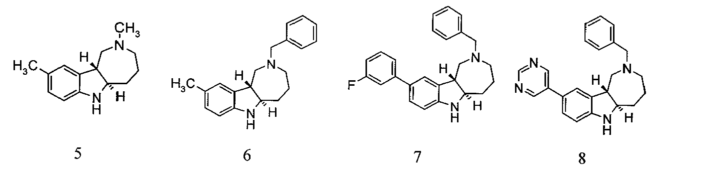

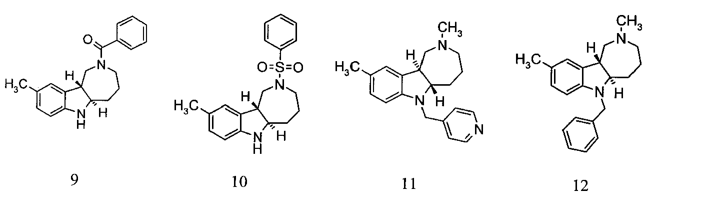

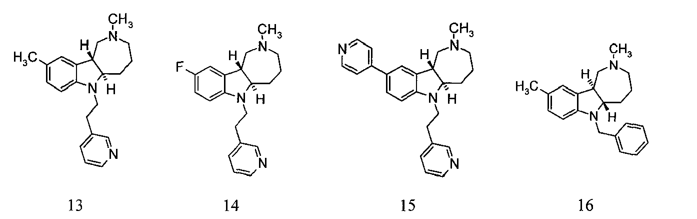

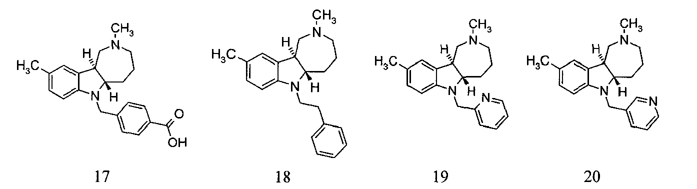

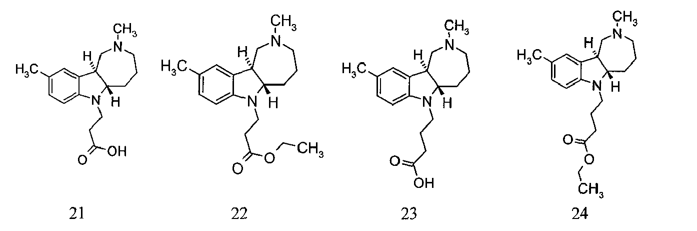

**Example 4.** Investigation of ligand activity of azaheterocyclic compounds of the general formula 1 to 5-HT₆ receptor. A focused library, including 3537 azaheterocyclic compounds of the general formula **1,** their geometric isomers and pharmaceutically acceptable salts were tested for ligand activity to 5-HT₆ receptor. Some examples of the tested azaheterocyclic compounds of the general formula 1 are represented in Tables 1-3, including: pyrrolo[4,3-b]indoles **1.2, 1.3** (Table 1), γ-carbolines **1.5, 1.6, 1.7** (Table 2) and azepino[4,3-b]indoles **1.10, 1.12, 1.13** (Tables 3). Ligand activity was determined by the ability of azaheterocyclic compounds of the general formula **1** to displace concurrently tritium-labelled diethyl ester of lysergic acid ([³H]LSD), specifically bound to serotonin 5-HT₆ receptors, which are expressed in HeLa cells membranes [Monsma FJ Jr, Shen Y, Ward RP, Hamblin MW and Sibley DR (1993). Cloning and expression of a novel serotonin receptor with high affinity for tricyclic psychotropic drugs. Mol Pharmacol. 43:320-327]. Cells membranes were incubated with 1.5 nM [³H]LSD for 2 hr at 37°C without and in the presence of tested compounds of 10 mkM concentration in the medium comprising: 50 mM Tris-HCl, pH 7.4, 150 mM NaCl, 2 mM ascorbic acid and 0.001% of bovine serum albumin. Nonspecific binding of [³H]LSD was determined in the presence of 5 mkM of serotonin (5-HT). After incubation membrane suspensions were filtered through glass microfiber filters GF/A (Millipor, USA) and the residual radioactivity was measured by scintillation counter Microbeta 1840 (PerkinElmer). Investigated compounds interacted effectively with 5-HT₆ receptor. Table 4 shows interaction efficiency of some of the tested compounds of the general formula 1 with 5-HT₆ receptor.

**Table 4. Examples of interaction efficiency of azaheterocyclic compounds of the general formula 1 to 5-HT₆ receptor.**

| N_{º} of azaheterocyclic compound of the general formula 1 | Interaction efficiency of compounds of the general formula 1 with 5-HT₆ receptor, % |
|---|---|
| **1.2(39)** | 77 |
| **1.3(9)** | 27 |
| **1.5(18)** | 71 |
| **1.5(20)** | 100 |
| **1.6(6)** | 52 |
| **1.6(22)** | 82 |
| **1.7(1)** | 47 |
| **1.10(49)** | 88 |
| **1.10(50)** | 84 |
| **1.10(51)** | 84 |
| **1.10(53)** | 79 |
| **1.12(12)** | 84 |
| **1.12(13)** | 76 |
| **1.13(3)** | 93 |
| **1.13(13)** | 93 |

**Example 5.** Measurements were carried out as it is described in example 1, except that the tested compounds were taken in various concentrations and % of displacement of [³H]LSD was plotted as a function of compound's concentration (fig. 1), on the basis of which IC₅₀ (concentration of halfmaximum blocking of [³H]LSD binding) values characterizing the seeming affinity of substituted azaheterocyclic compounds of the general formula 1 to 5-HT₆ receptor were calculated (Table 5).

**Table 5. The values of seeming affinity of substituted azaheterocyclic compounds of the general formula 1 to 5-HT₆ receptor.**

| Compound | IC₅₀, µM |
|---|---|
| **1.5(20)** | 0.074 |
| **1.10(50)** | 1.250 |
| **1.10(51)** | 1.060 |
| **1.10(53)** | 0.738 |
| **1.13(13)** | 0.787 |

**Example 6.** An example illustrating the preparation of tablets comprising 100 mg of an active ingredient. Mix together 1600 mg of starch, 1600 mg of grained lactose, 400 mg of talcum and 100 mg of azaheterocyclic compound **1.5(20)** or **1.10(49)** and press together in a brick. Prepared brick was crushed to granules and riddled through sieves, gathering granules of 14-16 mesh size. The obtained granules were pelletised in tablets of suitable form of 560 mg by weight each. According to the invention medicaments in the form of tablets comprising as an active ingredient other azaheterocyclic compounds of the general formula 1 could be prepared in a similar way.

**Example 7.** According to the invention capsules comprising 200 mg of azaheterocyclic compound **1.5(20),** or **1.10(49),** or **1.10(53)** could be prepared by careful mixing of compound **1.5(20),** or **1.10(49),** or **1.10(53)** with powder of lactose in ratio 2 : 1. The prepared powdery mixture is packed on 300 mg into gelatinous capsule of suitable size.

**Example 8.** Injectable compositions for intramuscular, intraperitoneal or hypodermic injections could be prepared by mixing 500 mg of an active ingredient with proper solvents, for example, hydrochloride of azaheterocyclic compound **1.10(53)** with 300 mg of chlorobutanole, 2 ml of propylene glycol and 100 ml of water for injections. The prepared solution was filtered and placed in 1 ml ampoules which were sealed up and sterilized in an autoclave.

### Industrial applicability

The invention could be used in medicine, veterinary, biochemistry.

## Claims

1. Use of azaheterocyclic compounds of the general formula **1** either racemates or optical isomers, or geometrical isomers, or pharmaceutically acceptable salts and/or hydrates thereof as ligands of 5-HT₆ receptor, 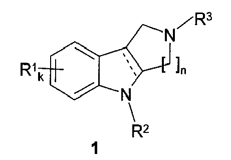 Wherein:
R² and R³ independently of each other represent amino group substituents selected from hydrogen; substituted carbonyl; substituted aminocarbonyl; substituted aminothiocarbonyl; substituted sulfonyl; C₁-C₅-alkyl optionally substituted with C₆-C₁₀-aryl, optionally substituted heterocyclyl, C₆-C₁₀-arylaminocarbonyl, C₆-C₁₀-arylaminothiocarbonyl, C₅-C₁₀-azaheteroaryl, optionally substituted carboxyl, CN-group; optionally substituted aryl;
R¹ₖ represents from 1 to 3 substituents of cyclic system independent of each other and selected from hydrogen, optionally substituted C₁-C₅-alkyl, C₁-C₅-alkoxy, C₁-C₅-alkenyl, C₁-C₅-alkynyl, halogen, trifluoromethyl, CN-group, carboxyl, optionally substituted aryl, optionally substituted heterocyclyl, substituted sulfonyl, optionally substituted carboxyl; the solid line accompanied by the dotted line, i.e. (---), represents a single or a double bond; n= 1, 2 or 3.

2. Use as claimed in claim 1, **characterized in that** substituted pyrrolo[4,3-b]indoles of the general formula **1.1** are used as azaheterocyclic compounds, 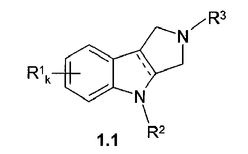 Wherein:
R¹ₖ, R², R³ and the solid line accompanied by the dotted line (---) are all as defined above.

3. Use as claimed in claim 2, **characterized in that** substituted 1,2,3,4-tetrahydropyrrolo[4,3-b]indoles of the general formula **1.2** are used as azaheterocyclic compounds, 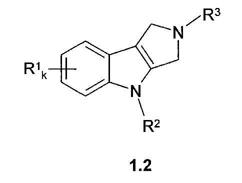 Wherein:
R¹ₖ, R² and R³ are all as defined above.

4. Use as claimed in claim 2, **characterized in that** substituted *cis*-1,2,3,3a,4,8b-hexahydropyrrolo[4,3-b]indoles of the general formula **1.3** are used as azaheterocyclic compounds, 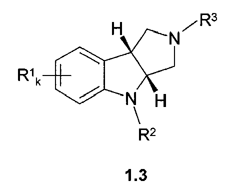 wherein:
R¹ₖ, R² and R³ are all as defined above.

5. Use as claimed in claim 1, **characterized in that** substituted γ-carbolines of the general formula **1.4** are used as azaheterocyclic compounds, 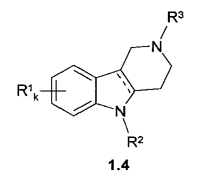 Wherein:
R¹ₖ, R², R³ and the solid line accompanied by the dotted line (---) are all as defined above.

6. Use as claimed in claim 5, **characterized in that** substituted 2,3,4,5-tetrahydro-γ-carbolines of the general formula **1.5** are used as azaheterocyclic compounds, 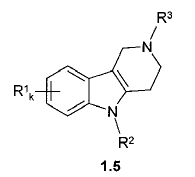 Wherein:
R¹ₖ, R² and R³ are all as defined above.

7. Use as claimed in claim 5, **characterized in that** substituted 2,3,4,4a,5,9b-hexahydro-γ-carbolines of the general formula **1.6** are used as azaheterocyclic compounds, 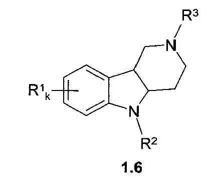 Wherein:
R¹ₖ, R² and R³ are all as defined above.

8. Use as claimed in claim 5, **characterized in that** differs in the fact that substituted *cis-*2,3,4,4a,5,9b-hexahydro-γ-carbolines of the general formula **1.7** and *trans*-2,3,4,4a,5,9b-hexahydro-γ-carbolines of the general formula **1.8** are used as azaheterocyclic compounds, 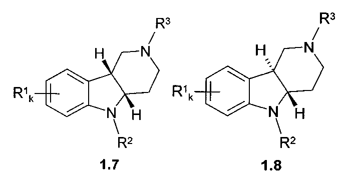 Wherein:
R¹ₖ, R² and R³ are all as defined above.

9. Use as claimed in claim 1, **characterized in that** substituted azepino[4,3-b]indoles of the general formula **1.9** are used as azaheterocyclic compounds, 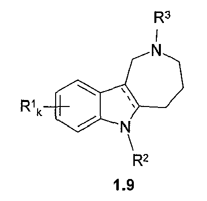 Wherein:
R¹ₖ, R², R³ and the solid line accompanied by the dotted line (---) are all as defined above.

10. Use as claimed in claim 9, **characterized in that** substituted 1,2,3,4,5,6-hexahydroazepino[4,3-b]indoles of the general formula **1.10** are used as azaheterocyclic compounds, 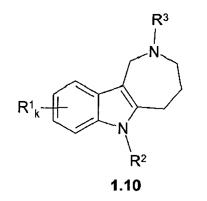 Wherein:
R¹ₖ, R² and R³ are all as defined above.

11. Use as claimed in claim 9, **characterized in that** substituted 1,2,3,4,5,5a,6,10b-octahydroazepino[4,3-b]indoles of the general formula **1.11** are used as azaheterocyclic compounds, 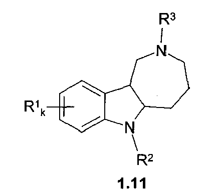 Wherein:
R¹ₖ, R² and R³ are all as defined above.

12. Use as claimed in claim 9, **characterized in that** substituted *cis*-1,2,3,4,5,5a,6,10b-octahydroazepino[4,3-b]indoles of the general formula **1.12** are used as azaheterocyclic compounds, 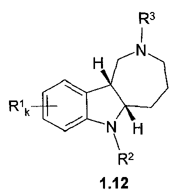 Wherein:
R¹ₖ, R² and R³ are all as defined above.

13. Use as claimed in claim 9, **characterized in that** substituted *trans*-1,2,3,4,5,5a,6,10b-octahydroazepino[4,3-b]indoles of the general formula **1.13** are used as azaheterocyclic compounds, 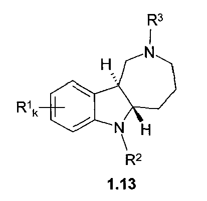 Wherein:
R¹ₖ, R² and R³ are all as defined above.

14. A pharmaceutical composition for preparation of medicament intended for prophylaxis and treatment of various diseases and conditions of central nervous system (CNS) pathogenesis of which is associated with 5-HT₆ receptors, comprising as an active ingredient pharmaceutically effective amount of ligand as claimed in any of claims 1-13 with the exception of:
compounds of the general formula **A:** 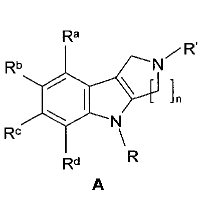 wherein:
n =1; R^{a} = R^{b} = R^{c}=R^{d}= H; R'= CH₃;
n =1; R^{a} = R^{b} = R^{c}=R^{d}= H; R'= n-butyl;
n = 2; R^{a} = R^{b} = R^{c}=R^{d}= H; R'= CH₃;
n = 2; R^{a} = R^{b} = R^{c}=R^{d}= H; R'= C₂H₅;
n = 2; R^{a} = R^{b} = R^{c}=R^{d}= H; R'= benzyl;
n = 2; R^{a} = = R^{c}=R^{d}= H; R'= R^{b}= CH₃;
n = 2; R^{a} = = R^{c}=R^{d}= H; R'= CH₃; R^{b}= Br;
n = 2; R^{a} = R^{c}=R^{d}= H; R^{b}=CH₃, R= 2-(6-methylpyridin-3-yl)ethyl;
n = 2; R^{a} = = R^{c}=R^{d}= H; R=H; R'= CH₃; R^{b}= CH(CH₃)₂;
n = 2; R^{a} = R^{c}=R^{d}= H; R'= R^{b}= CH₃; R= H; 2-(6-methylpyridin-3-yl)ethyl; benzyl; 2,4-dimethylbenzyl, 2-piperidinoethyl; 2-hexamethyleneiminoethyl; 2-cyanoethyl; 2-carboxyethyl; 2-carbethoxyethyl; [5-methyl-2-(2-N,N-dimethylaminoethyl)indol-3-ylmethyl]; methylcarbamoyl;
n =3; R= H, R^{a} = R^{b} = R^{c}=R^{d}= H, R'= phenylaminocarbonyl; 2-chlorophenylaminocarbonyl; 2-fluorophenylaminocarbonyl; 3-methylphenylaminocarbonyl,
and compounds of the general formula **B:** 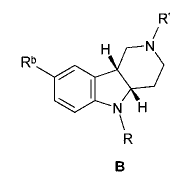 wherein:
R^{b}= H, R'=CH₃; 2-(6-methylpyridin-3-yl)ethyl;
R^{b}= R'=CH₃; R = H;
R^{b}=R'= CH₃; R = carbamoyl; thiocabamoyl; phenylthiocarbamoyl; cyclohexylthiocarbamoyl; n-butylcarbamoyl; phenylcarbamoyl; trifluoroacetyl; 2-bromobenzoyl; 2-methylbenzoyl; 3,4,5-trimethoxybenzoyl; 1-naphthylacetyl; methylsulfonyl; 2-nitrophenylsulfonyl; (pyridin-3-yl)sulfonyl; 2-bromopropionyl; chloroacetyl; (E)-2-butenoyl; methoxyacetyl; N-pyrrolidinoacetyl; morpholinoacetyl; 2-N-piperidinopropionyl; 2-N-morpholinopropionyl; 4-methyl-1-pyridiniumacetyl; 1-pyridiniumacetyl; 2-(4-methyl-1-pyridinium)propionyl; 3-carboxypropionyl,
and also compounds of the general formula **C:** 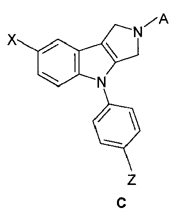 wherein:
A = C₁-C₆ is a straight or branched alkyl possibly substituted with phenyl, presumably p-substituted with H, Cl, F.
X = H, F, Cl, Br, CH₃;
Z = H, F, Cl, OCH₃.

15. Use at least one ligand as claimed in any of claims 1-13 or a pharmaceutical composition as claimed in claim 14 for preparation of a medicament in the form of tablets, capsules or injections, placed in a pharmaceutically acceptable packing for treatment and prophylaxis of diseases and conditions of central nervous system (CNS) pathogenesis of which is associated with 5-HT₆ receptors.
